(19) Europäisches Patentamt
European Patent Office
Office européen des brevets

(11) Publication number: **0 322 633 B1**

# (12) EUROPEAN PATENT SPECIFICATION

(45) Date of publication of patent specification: **22.05.91 Bulletin 91/21**

(51) Int. Cl.$^5$: **C07C 327/02**, C07C 321/02, A61K 31/16

(21) Application number: **88120795.5**

(22) Date of filing: **13.12.88**

## (54) Mercapto-acylamino acid antihypertensives.

Consolidated with 89900561.5/0390839 (European application No./publication No.) by decision dated 08.11.90.

(30) Priority: **16.12.87 US 133669**

(43) Date of publication of application: **05.07.89 Bulletin 89/27**

(45) Publication of the grant of the patent: **22.05.91 Bulletin 91/21**

(84) Designated Contracting States: **AT BE CH DE ES FR GB GR IT LI LU NL SE**

(56) References cited:
**EP-A- 0 038 758**
**EP-A- 0 110 224**
**CH-A- 637 374**
**US-A- 4 173 704**
**US-A- 4 401 677**

(73) Proprietor: **SCHERING CORPORATION**
**2000 Galloping Hill Road**
**Kenilworth New Jersey 07033 (US)**

(72) Inventor: **Haslanger, Martin F.**
**53 W. Glen Avenue**
**Ridgewood, NJ 07450 (US)**
Inventor: **Neustadt, Bernard Ray**
**24 Brook Place**
**West Orange, NJ 07052 (US)**
Inventor: **Smith, Elizabeth Melva**
**166 Grove Avenue**
**Verona, NJ 07044 (US)**

(74) Representative: **von Kreisler, Alek, Dipl.-Chem. et al**
**Patentanwälte Von Kreisler-Selting-Werner,**
**Deichmannhaus am Hauptbahnhof**
**W-5000 Köln 1 (DE)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).



Jouve, 18, rue Saint-Denis, 75001 PARIS

**Description**

Human hypertension represents a disease of multiple etiologies. Included among these is a sodium and volume dependent low renin form of hypertension. Drugs that act to control one aspect of hypertension will not necessarily be effective in controlling another.

A variety of mercapto-acylamino acids are known as enkephalinase inhibitors useful as analgesics and in the treatment of hypertension.

U.S. 4,513,009 to Roques et al discloses, inter alia, compounds of the formula.

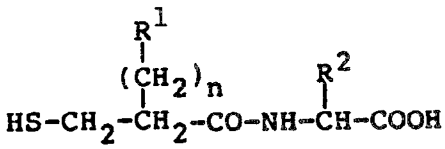

wherein n is 0 to 1 ; $R^1$ includes hydrogen, optionally substituted alkyl, optionally substituted phenyl, cyclohexyl and thienyl ; and $R^2$ includes hydrogen optionally substituted alkyl, optionally substituted benzyl, phenyl, phenoxyalkyl and optionally substituted mercaptoalkyl. The compounds are disclosed as principally having enkephalinase activity, but also are said to be antihypertensives.

U.S. 4,401,677 to Greenberg et al and EPA 38,046 to Wilkinson disclose compounds of a scope similar to Roques et al, the former disclosing analgesic activity and the latter disclosing a greater specificity for enkephalinase than ACE. U.S. 4,053,651 to Ondetti et al discloses the use of similar compounds in the treatment of renin-angiotensin related hypertension.

It has recently been discovered that the heart secretes a series of peptide hormones called atrial natriuretic factors (ANF) which help to regulate blood pressure, blood volume and the excretion of water, sodium and potassium. ANF were found to produce a short-term reduction in blood pressure and to be useful in the treatment of congestive heart failure. See P. Needleman et al, "Atriopeptin : A Cardiac Hormone Intimately Involved in Fluid, Electrolyte and Blood-Pressure Homeostasis", N. Engl. J. Med., 314, 13 (1986) pp. 828-834, and M. Cantin et al in "The Heart as an Endocrine Gland", Scientific American, 254 (1986) pg. 76-81.

A class of drugs known to be effective in treating some types of hypertension is ACE inhibitors, which compounds are useful in blocking the rise in blood pressure caused by increases in vascular resistance and fluid volume due to the formation of angiotensin II from angiotensin I. For a review of ACE inhibitors, see M. Wyvratt and A. Patchett, "Recent Developments in the Design of Angiotensin Converting Enzyme Inhibitors" in Med. Res. Rev. Vol. 5, No. 4 (1985) pp. 483-531.

The present invention relates to mercapto-acylamino acids useful in the treatment of various types of hypertension, particularly volume expanded hypertension, and congestive heart failure.

These novel compounds have been found to enhance both the magnitude and duration of the antihypertensive and natriuretic effects of endogenous ANF. Administration of a combination of a mercapto-acylamino acid and an ACE inhibitor provides an antihypertensive effect greater than either the mercapto-acylamino acid or ACE inhibitor alone. Administration of a combination of a mercapto-acylamino acid of the present invention and an exogenous ANF or ACE inhibitor is therefore particularly useful in treating hypertension.

In addition to the compound aspect, the present invention therefore also relates to treating hypertension with a mercapto-acylamino acid or with a mercapto-acylamino acid in combination with an ANF or an ACE inhibitor, which methods comprise administering to a mammal in need of such treatment an antihypertensive effective amount of the mercapto-acylamino acid or an antihypertensive effective amount of a combination of a mercapto-acylamino acid and ANF or ACE inhibitor. The drug or combination of drugs is preferably administered in a pharmaceutically acceptable carrier, e.g. for oral or parenteral administration. The combinations of drugs may be co-administered in a single composition, or components of the combination therapy may be administered separately. Where the components are administered separately, any convenient combination of dosage forms may be used, e.g. oral mercapto-acylamino acid/oral ANF, oral mercapto-acylamino acid/parenteral ACE inhibitor, parenteral mercapto-acylamino acid/oral ANF, parenteral mercapto-acylamino acid/parenteral ACE inhibitor.

When the components of a combination of a mercapto-acylamino acid and an ANF are administered separately, it is preferred that the mercapto-acylamino acid be administered first.

Another aspect of the invention relates to pharmaceutical compositions comprising a mercapto-acylamino acid of this invention, alone or in combination with an ANF or an ACE inhibitor, and to methods of treatment of hypertension and congestive heart failure comprising administering a mercapto-acylamino acid of this invention, alone or in combination with an ANF or an ACE inhibitor to a mammal in need of such treatment.

Novel mercapto-acylamino acid antihypertensive compounds of the present invention are represented by

the following formula :

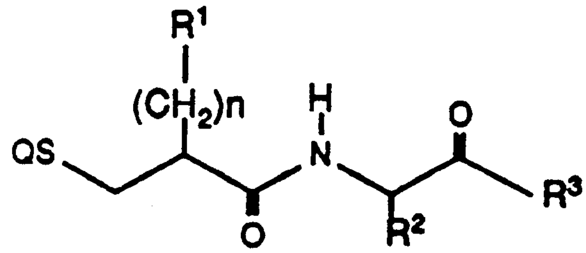

I

wherein $R^1$ is $Y\text{-}C_6H_4-$, $Y\text{-}C_6H_4S-$, $Y\text{-}C_6H_4O-$,

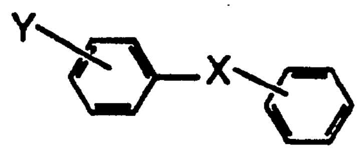

α-naphthyl, ß-naphthyl, furyl, thienyl, benzofuryl, benzothienyl, $H_2N(CH_2)_m$-, diphenylmethyl,

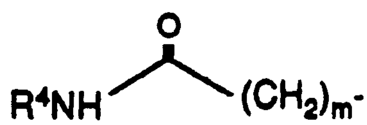

$R^2$ is $R^{14}(CH_2)_kS(O)_{0-2}(CH_2)_q$- or $R^6OCO(CH_2)_q$- ; $R^3$ is $-OR^7$,

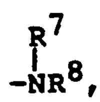, 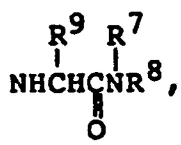, 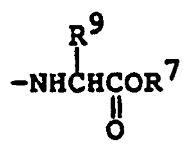 or 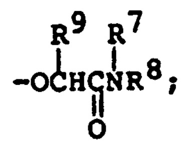;

$R^4$ is hydrogen, alkyl or $Y^1\text{-}C_6H_4-$ ;
$R^{14}$ is mono-unsaturated lower alkyl, hydroxy, alkoxy or alkylthio, provided that when $R^{14}$ is hydroxy or alkoxy, k is 2 or 3 and when $R^{14}$ is mono-unsaturated alkykl or alkylthio, k is 1, 2 or 3 ;
$R^6$ is dihydroxyalkyl, dialkoxyalkyl, alkoxyalkoxyalkyl, haloalkyl, (haloalkoxy)alkyl or alkyl substituted with a 5-6 membered saturated ring comprising 1-2 oxygen atoms as ring members wherein the ring carbon atoms may be substituted with 0-2 alkyl substituents ;
$R^7$ and $R^8$ are independently $R^6$, H, alkyl, hydroxyalkyl, alkoxyalkyl, aminoalkyl, alkylaminoalkyl, dialkylaminoalkyl or arylalkyl, or $R^7$ and $R^8$ together with the nitrogen to which they are attached complete a 5-7 membered ring, wherein one of the 4-6 ring members comprising $R^7$ and $R^8$ may be a nitrogen atom, an alkyl-substituted nitrogen atom or an oxygen atom, and wherein the ring may be substituted on the ring carbon atoms with substituents chosen from alkyl and hydroxy groups ;
$R^9$ is hydrogen, alkyl, carboxyalkyl, mercaptoalkyl, alkylthioalkyl, aminoalkyl, hydroxyalkyl, phenylalkyl, hydroxyphenylalkyl, guanidinoalkyl, imidazolylalkyl, indolylalkyl or carbamoylalkyl ;
n is 0-2 ;
m and k are independently 0-3 ;
q is 1-4 ;
X is a bond, –O–, –S–, or $-CH_2-$ ;
Q is hydrogen or $R^{10}CO-$ ;
$R^{10}$ is alkyl, hydroxyalkyl, alkoxyalkyl, dialkylaminoalkyl, $Y^2\text{-}C_6H_4$-alkyl, alkoxy, $Y^2\text{-}C_6H_4-$, naphthyl, furyl, thienyl

or pyridyl ;
Y, $Y^1$ and $Y^2$ independently represent one or more substituents selected from H, alkyl, cycloalkyl, alkoxy, OH, F, Cl, Br, CN, $-CH_2NH_2$, $-CO_2H$, $-CO_2$alkyl, $-CONH_2$ and phenyl ; the pharmaceutically acceptable addition salt thereof.

As used herein the term "alkyl" means straight or branched alkyl chains of 1 to 6 carbon atoms, and "alkoxy" similarly refers to alkoxy groups having 1 to 6 carbon atoms. "Cycloalkyl" means cyclic alkyl groups of 3-6 carbon atoms.

"Aryl" means mono-cyclic or fused ring bi-cyclic carbocyclic aromatic groups having 6 to 10 ring members or mono-cyclic or fused ring bycyclic aromatic groups wherein 1-2 ring members may independently be nitrogen, oxygen or sulfur, wherein the carbon ring members of the aryl group are substituted by 0-3 substituents as defined above by Y. Examples of carbocyclic aryl groups are phenyl, α-naphthyl and ß-naphthyl, and examples of heterocyclic aryl groups are furyl, thienyl, benzofuryl, benzothienyl, indolyl and pyridyl. All positional isomers, e.g. 2-pyridyl, 3-pyridyl, are contemplated.

"Halo" refers to fluorine, chlorine, bromine or iodine radicals. The term "poly", when used to describe substitution in a phenyl, alkylphenyl or alkoxyphenyl group, means 2 to 5 substituents.

Groups $R^3$ comprising the partial structure

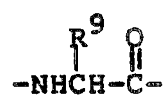

are derived from amino acids of formula

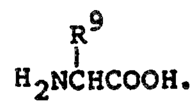

Examples of such amino acids are alanine, arginine, asparagine, aspartic acid, cysteine, glutamine, glutamic acid, glycine, histidine, isoleucine, leucine, lysine, methionine, phenylalanine, serine, threonine, tryptophan, tyrosine and valine.

Preferred embodiments of compounds of formula I are compounds wherein $R^1$ is $Y-C_6H_4-$ especially wherein Y is hydrogen. Also preferred are compounds wherein $R^3$ is $-OR^7$ or $-NR^7R^8$, wherein $R^7$ and $R^8$ are as defined above. Further preferred compounds of formula I are those wherein Q is hydrogen or $R^{10}CO-$ wherein $R^{10}$ is alkyl, especially methyl, or phenyl. Also preferred are compounds wherein $R^2$ is $R^{14}(CH_2)_k-(S(O)_{0-2}(CH)_q-$. Especially preferred $R^{14}$ groups are mono-unsaturated lower alkyl such as vinyl and alkylthio such as methylthio.

Compounds of this invention may, depending on the nature of functional groups, form addition salts with various inorganic and organic acids and bases. Such salts include salts prepared with organic and inorganic acids, e.g. HCl, HBr, $H_2SO_4$, $H_3PO_4$, methanesulfonic acid, toluenesulfonic acid, maleic acid, furmaric acid and camphorsulfonic acid. Salts prepared with bases include ammonium salts, alkali metal salts, e.g. sodium and potassium salts, and alkaline earth salts, e.g. calcium and magnesium salts.

The salts may be formed by conventional means, as by reacting the free acid or base forms of the product with one or more equivalents of the appropriate base or acid in a solvent or medium in which the salt is insoluble, or in a solvent such as water which is then removed in vacuo or by freeze-drying or by exchanging the cations of an existing salt for another cation on a suitable ion exchange resin.

Compounds of formula I have two or more asymmetrical carbon atoms and therefore include various stereoisomers. All stereoisomers are included within the scope of the present invention.

In general, compounds of the present invention can be made by an appropriate process selected from the following process A and B, wherein Q, $R^1$, $R^2$, $R^3$ and n are as defined in claim 1, including suitable protection:

Process A : condensation of a 3-thiopropionic acid of formula IV, or a reactive derivative thereof, with an amine of formula V

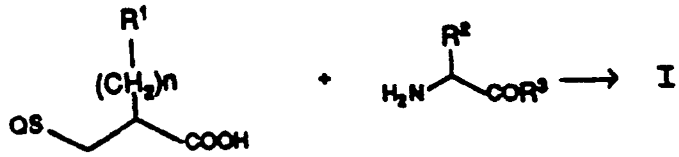

IV V

Process B : for compounds of formula I wherein $R^3$ is $-NR^7R^8$, condensation of a (3-thiopropionyl)amino acid of formula VI, or a reactive derivative thereof, with an amine of formula VII

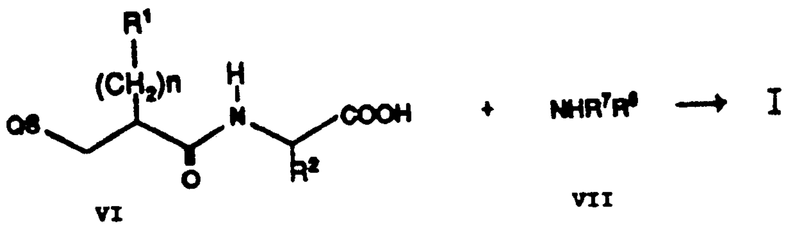

VI VII

wherein both Process A and Process B are followed by isolating the preferred isomer if desired and removal of the protecting groups, if necesary, to yield the desired products, and if desired, preparing a salt thereof.

More particularly, compounds of the present invention may be prepared by using coupling reactions well known in the peptide art to join a 3-acetylthio-2-(substituted)-propionic acid of formula 1 with an amino acid ester of formula 2. The following reaction Scheme 1 is an example :

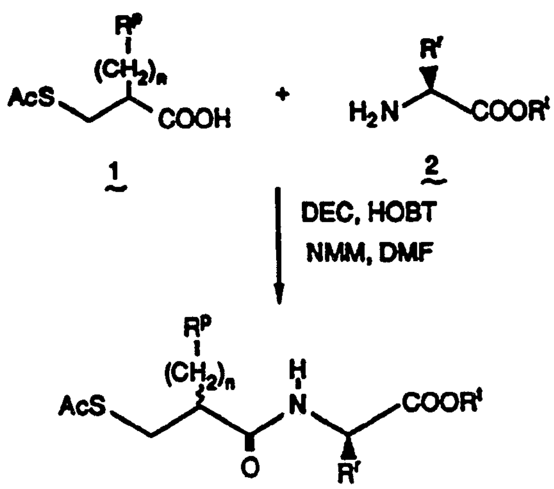


1 2

3

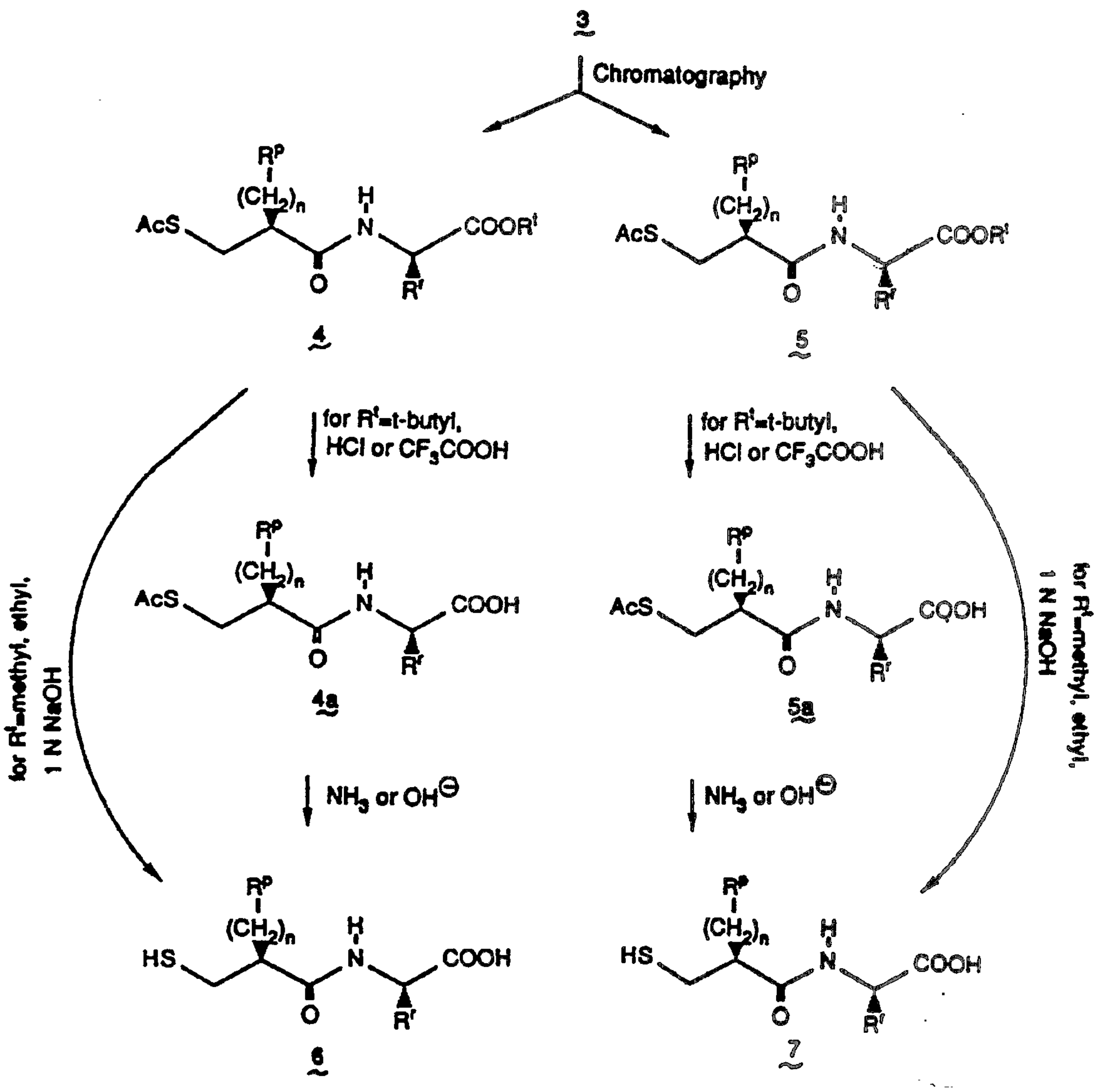


In the above scheme, $R^p = R^1$ ; $R^r = R^2$ ; $R^t$ is methyl, ethyl, t-butyl or aralkyl (e.g. benzyl) ; Ac is acetyl ; n is 0-2 ; DEC is 1-(3-dimethylaminopropyl)-3-ethylcarbodiimide hydrochloride ; HOBT is 1-hydroxybenzotriazole hydrate ; NMM is N-methylmorpholine ; and DMF is dimethylformamide.

As Scheme 1 shows, an amino acid ester of formula 2 and a 3-acetylthio propionic acid of formula 1 are reacted at room temperature in an inert solvent such as DMF in the presence of coupling agents such as DEC and HOBT in the presence of a base such as NMM. The resultant isomers are separated by chromatography and the isomers are deprotected at the acid and mercapto positions.

Alternatively, a propionic acid of formula 1 may be reacted with thionyl chloride to prepare the corresponding propionyl chloride, which may then be reacted with an amino acid ester of formula 2 or with the corresponding free acid 2a in an inert solvent such as acetonitrile in the presence of a base such as triethylamine to give isomers of formula 3, which may be separated as in Scheme 1. The following Scheme 2 is an example :

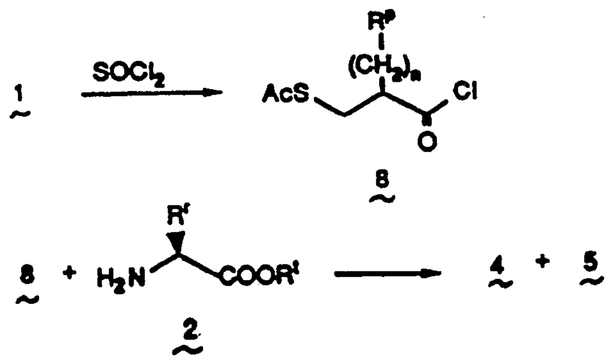


1

8

8 + 2 → 4 + 5

2

wherein n, Ac, $R^p$, $R^r$ and $R^t$ are as defined above, and wherein $R^t$ may also be hydrogen.

Other $R^3$ esters of compounds of formula I are prepared by standard esterification techniques, for example N-(t-butoxycarbonyl)-S-allyl-(R)-cysteine is reacted with 2- (2-chloroethoxy)ethanol in the presence of a coupling agent such as DEC and a base such as 4-dimethylaminopyridine, the amino function is deprotected and the resultant amino acid ester is reacted with a compound of formula 1 in a manner similar to that described in Scheme 2. In another example, N-(t-butoxycarbonyl)-S-methylthiomethyl-(R)-cysteine is reacted with N,N-diethylbromoacetamide and a reagent such as cesium carbonate, the resultant ester is deprotected at the amino function and a reaction similar to that described in Scheme 2 is carried out.

Compounds of formula I wherein $R^3$ is $-NR^7R^8$ are prepared by coupling reactions as described above in Schemes 1 and 2 by replacing the amino acid ester 2 with an amide or substituted amide as shown in Scheme 3 :

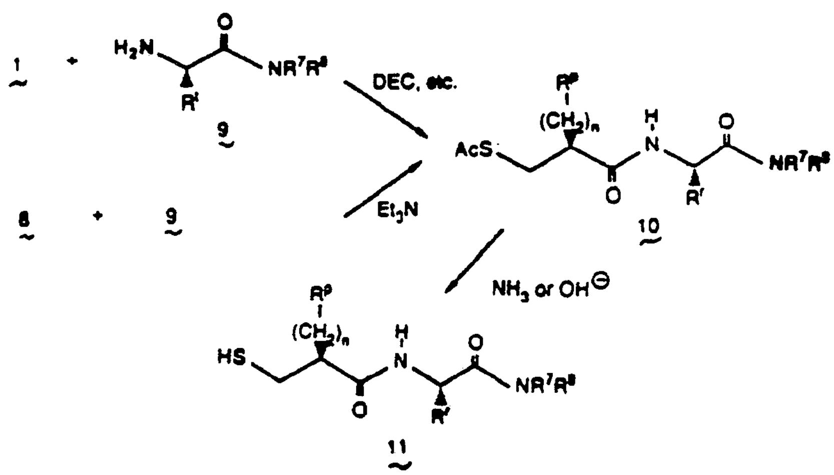


1 + 9

9

8 + 9

10

11

Alternatively, compounds of formula I wherein $R^3$ is $-NR^7R^8$ may be prepared by coupling a propionyl chloride of formula 8 with an amino acid of formula 2a in the presence of a base and then coupling the desired $-NR^7R^8$ group to the carboxylic group using a typical peptide-coupling reaction. Scheme 4 shows an example of such a procedure :

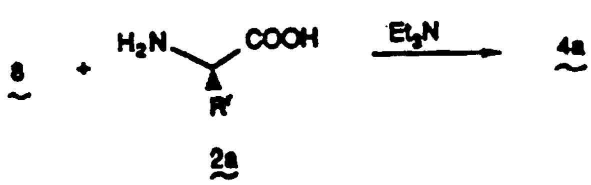


8 + 2a → 4a

2a

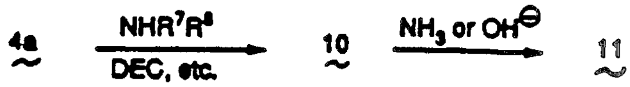


A third method for preparing compounds of formula I wherein $R^3$ is $-NR^7R^8$ comprises reacting a propionic acid of formula 1 with an amino acid t-butyl ester of formula 2, removing the t-butyl ester and coupling the $-NR^7R^8$ group to the carboxylic acid group as above. Compounds wherein $R^3$ is

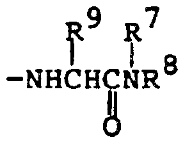 and 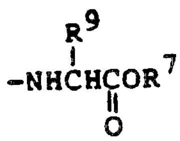

are prepared analogously to those wherein $R^3$ is $-NR^7R^8$.

Compounds wherein Q is $R^{10}CO-$ may be prepared by known methods, for example by adding a mercaptoacid of formula $R^{10}COSH$ to an acrylic acid to obtain a thio-substituted propionic acid analogous to compounds of formula 1. Alternatively, an amide of formula I wherein Q is $-SH$ may be reacted with a compound of formulae $R^{10}COCl$ in the presence of a base to obtain the desired sulfur substituted derivative.

Compounds wherein $R^2$ is $R^{14}(CH_2)_k\text{-}S(O)_{1\text{-}2}\text{-}(CH_2)_q-$ are prepared by oxidizing with hydrogen peroxide the corresponding substituted alkylthioalkyl compound of formula I (e.g. those wherein $R^2$ is, e.g. $R^{14}(CH_2)_k\text{-}S\text{-}(CH_2)_q-$).

Starting materials of formulae 1 and 2 are known in the art or may be prepared by methods well known to those skilled in the art. See for example U.S. 4,329,495 for the preparation of the R and S enantiomers of 3-benzoylthio-2-benzylpropionic acid.

A second aspect of the invention is the administration of a combination of a compound of formula I and an ANF.

As indicated by Needleman et al., a number of ANF have been isolated so far, all having the same core sequence of 17 amino acids within a cysteine disulfide bridge, but having different N-termini lengths. These peptides represent N-terminal truncated fragments (21-48 amino acids) of a common preprohormone (151 and 152 amino acids for man and rats, respectively). Human, porcine and bovine carboxy-terminal 28-amino acid peptides are identical and differ from similar peptides in rats and mice in that the former contain a methionine group at position 12 while the latter contain isoleucine. Various synthetic analogs of naturally occurring ANFs also have been found to have comparable biological activity. Examples of ANFs contemplated for use in this invention are α human AP 21 (atriopeptin I), α human AP 28, α human AP 23 (atriopeptin II or APII), α human AP 24, α human AP 25, α human AP 26, α human AP 33, and the corresponding rat sequence of each of the above wherein Met 12 is Ile. See Table 1 for a comparison of the peptides.

TABLE 1

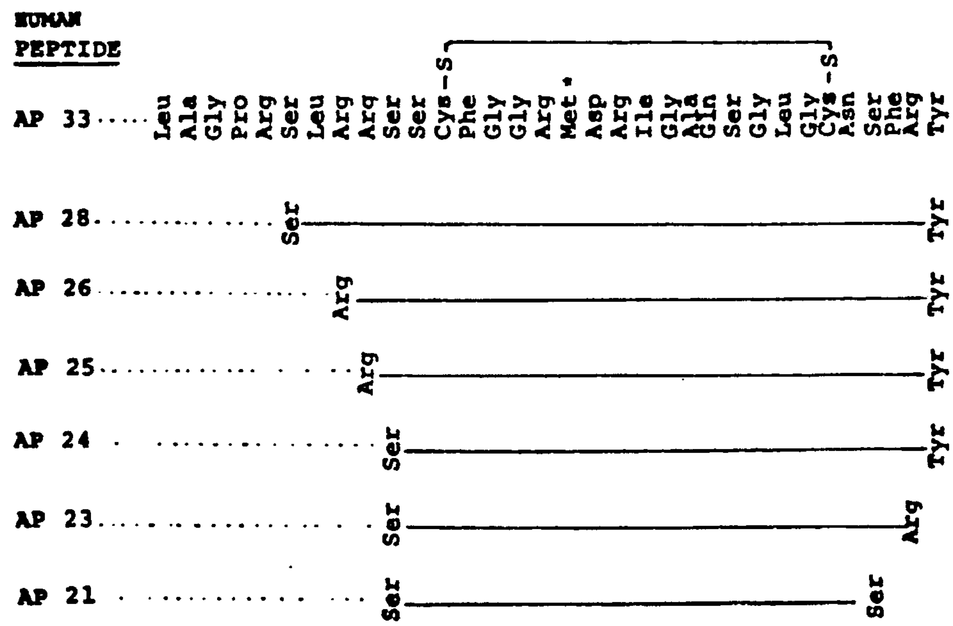


* Ile in the rat peptide

A third aspect of the invention in the administration of a combination of an ACE inhibitor and a compound of formula I.

Examples of ACE inhibitors are those disclosed in the article by Wyvratt et al., cited above, and in the following publications : U.S. Patents 4,105,776, 4,468,519, 4,555,506, 4,374,829, 4,462,943, 4,470,973, 4,470,972, 4,350,704, 4,256,761, 4,344,949, 4,508,729, 4,512,924, 4,410,520 and 4,374,847 ; and the following foreign patents or published patent applications : GB 2,095,682, EPA 50,800, EPA 79,522, EPA 79,022 and EPA 46,953.

Following are preferred ACE inhibitors for use in the combination of this invention : spirapril, enalapril, ramipril, perindopril, indolapril, lysinopril, pentopril, cilazapril, captopril, zofenopril, pivalopril, fosinopril and L-(5)-2-[N-[1-(ethoxycarbonyl)-3-phenylpropyl]alanyl]-1,2,3,4-tetrahydro-6,7-dimethoxyisoquinoline-3-carboxylic acid monohydrochloride.

The antihypertensive effect of mercapto-acylamino acids alone and in combination with ACE inhibitors is determined according to the following procedure :

Male Sprague Dawley rats weighing 100-150 g are anesthetized with ether and the right kidney is removed. Three pellets containing Doc acetate (desoxycorticosterone acetate, DOCA, 25 mg/pellet) are implanted subcutaneously. Animals recovered from surgery are maintained on normal rat chow and are allowed free access to a fluid of 1% NaCl and 0.2% KCl instead of tap water for a period of 17-30 days. This procedure results in a sustained elevation in blood pressure and is a slight modification of published procedures (e.g. Brock et al., 1982) that have been used to produce DOCA salt hypertension in the rat.

On the day of study, animals are again anesthetized with ether and the caudal artery is cannulated for blood pressure measurement. Patency of the caudal artery cannula is maintained with a continuous infusion of dextrose in water at a rate of 0.2 ml/hr. Animals are placed into restraining cages where they recover consciousness. Blood pressure is measured from caudal artery catheter using a Statham pressure transducer attached to a Beckman oscillographic recorder. In addition, a cardiovascular monitoring device (Buxco Electronics, Inc.) and a digital computer are used to calculate average blood pressures.

After an equilibration period of at least 1.5 hr., animals are dosed subcutaneously (1 ml/kg) with vehicle (methylcellulose, hereinafter MC) or mercapto-acylamino acid and blood pressure is monitored for the next 4 hours.

The antihypertensive effect of mercapto-acylamino acids in combination with ANF is determined according to the following procedures :

Male spontaneously hypertensive rats (SHR), 16-18 weeks old, 270-350 g, are anesthetized with ether and the abdominal aorta is cannulated through the tail artery. The animals were then placed into restrainers to recover from anesthesia (in less than 10 min.) and remain inside throughout the experiments. Through a pressure transducer (Gould P23 series) analog blood pressure signals are registered on a Beckman 612 recorder. A Buxco digital computer is used to obtain mean arterial pressures. Patency of the arterial cannula is maintained with a continuous infusion of 5% dextrose at 0.2 ml/hr. Animals are allowed a 90-min equilibration period. The animals first undergo a challenge with an ANF such as atriopeptin II (AP II) or AP28 30 µg/kg iv and at the end of 60 min. are treated with drug vehicle or a mercapto-acylamino acid subcutaneously. A second ANF challenge is administered 15 min. later and blood pressure is monitored for the next 90 min.

The antihypertensive effect in SHR of mercapto-acylamino acids and ACE inhibitors, alone and in combination, is determined as follows :

Animals are prepared for blood pressure measurement as described above. After stabilization, animals are dosed subcutaneously or orally with test drugs or placebo and blood pressure is monitored for the next 4 hr.

The compositions of this invention comprise a mercapto-acylamino acid or a mercapto-acylamino acid and an ANF or a mercapto-acylamino acid and an ACE inhibitor in combination with a pharmaceutically acceptable carrier for administration to mammals. A variety of pharmaceutical forms is suitable, preferably for oral or parenteral administration, although mechanical delivery systems such as transdermal dosage forms are also contemplated.

The daily antihypertensive dose of the compound or combinations of this invention is as follows : for mercapto-acylamino acids alone the typical dosage is 1 to 100 mg/kg of mammalian weight per day administered in single or divided dosages ; for the combination of mercapto-acylamino acid and an ANF, the typical dosage is 1 to 100 mg of mercapto-acylamino acid/kg mammalian weight per day in single or divided dosages plus 0.001 to 0.1 mg ANF/kg of mammalian weight per day, in single or divided dosages, and for the combination of mercapto-acylamino acid and an ACE inhibitor, the typical dosage is 1 to 100 mg of mercapto-acylamino acid/kg mammalian weight per day in single or divided dosages plus 0.1 to 30 mg ACE inhibitor/kg of mammalian weight per day in single or divided dosages. The exact dose of any component or combination to be administered is determined by the attending clinician and is dependent on the potency of the compound administered, the age, weight, condition and response of the patient. Compounds of this invention are not toxic at the therapeutically effective dose.

Generally, in treating humans having hypertension, the compounds or combinations of this invention may be administered to patients in a dosage range as follows : for treatment with mercapto-acylamino acids alone, about 10 to about 500 mg per dose given 1 to 4 times a day, giving a total daily dose of about 10 to 2000 mg per day ; for the combination of mercapto-acylamino acid and ANF, about 10 to about 500 mg mercapto-acylamino acid per dose given 1 to 4 times a day and about 0.001 to about 1 mg ANF given 1 to 6 times a day (total daily dosage range of 10 to 2000 mg day and.001 to 6 mg/day, respectively) ; and for the combination of a mercapto-acylamino acid and an ACE inhibitor, about 10 to about 500 mg mercapto-acylamino acid per dose given 1 to 4 times a day and about 5 to about 50 mg ACE inhibitor given 1 to 3 times a day (total daily dosage range of 10 to 2000 mg/day and 5 to 150 mg/day, respectively). Where the components of a combination are administered separately, the number of doses of each component given per day may not necessarily be the same, e.g. where one component may have a greater duration of activity, and will therefore need to be administered less frequently.

Typical oral formulations include tablets, capsules, syrups, elixirs and suspensions. Typical injectable formulations include solutions and suspensions. Typical pharmaceutically acceptable carriers may be used in said formulations.

Examples of formulations follow. "Drug" refers to any mercapto-acylamino acid of the present invention. "ACE inhibitor" refers to any of the ACE inhibitors listed on page 14, especially those in the list of preferred ACE inhibitors. "Atrial peptide" refers to any antihypertensive atrial peptide, especially those listed in Table 1.

EXAMPLE 1

Tablets

Formula

| No. | Ingredient | mg/tablet | mg/tablet |
|---|---|---|---|
| 1 | Drug | 50 | 400 |
| 2 | Lactose | 122 | 213 |
| 3 | Corn Starch, Food Grade, as a 10% paste in Purified Water | 30 | 40 |
| 4 | Corn Starch, Food Grade | 45 | 40 |
| 5 | Magnesium Stearate | 3 | 7 |
| | Approximate Tablet Weight | 250 | 700 |

Method of Manufacture

Mix Item Nos. 1 and 2 in a suitable mixer for 10-15 minutes. Granulate the mixture with Item No. 3. Mill the damp granules through a coarse screen (e.g., 1/4") if needed. Dry the damp granules. Screen the dried granules if needed and mix with Item No. 4 and mix for 10-15 minutes. Add Item No. 5 and mix for 1-3 minutes. Compress the mixture to appropriate size and weight on a suitable tablet machine.

EXAMPLE 2

Drug for Injection : (per vial)

| | g/vial | g/vial |
|---|---|---|
| Drug: Sterile Powder | 0.5 | 1.0 |

Add sterile water for injection or bacteriostatic water for injection for reconstitution.

EXAMPLE 3

Drug for Injectable Solution :

| Ingredient | mg/ml | mg/ml |
|---|---|---|
| Drug | 100 | 500 |
| Methylparaben | 1.8 | 1.8 |
| Propylparaben | 0.2 | 0.2 |
| Sodium Bisulfite | 3.2 | 3.2 |
| Disodium Edetate | 0.1 | 0.1 |
| Sodium Sulfate | 2.6 | 2.6 |
| Water for Injection q.s. ad | 1.0 ml | 1.0 ml |

Method of Manufacture
Dissolve parabens in a portion (85% of the final volume) of the water for injection at 65-70°C. Cool to 25-35°C. Charge and dissolve the sodium bisulfite, disodium edetate and sodium sulfate. Charge and dissolve the drug. Bring the solution to final volume by adding water for injection. Filter the solution through 0.22μ membrane and fill into appropriate containers. Terminally sterilize the units by autoclaving.

EXAMPLE 4

Drug for Injection : (per vial)

| | g/vial |
|---|---|
| Drug | 1.0 |
| Sodium Citrate | 0.05 |

pH is adjusted to 6.2 using 0.1N citric acid solution.
Add sterile water for injection or bacteriostatic water for injection for reconstitution.

EXAMPLE 5

Tablets

Formula

| No. | Ingredient | mg/tablet | mg/tablet |
|---|---|---|---|
| 1 | Drug | 50 | 400 |
| 2 | ACE inhibitor | 25 | 50 |
| 3 | Lactose | 97 | 188 |
| 4 | Corn Starch, Food Grade as a 10% Paste in Purified Water | 30 | 40 |
| 5 | Corn Starch, Food Grade | 45 | 40 |
| 6 | Magnesium Stearate | 3 | 7 |
| | Approximate Tablet Weight | 250 | 700 |

Method of Manufacturing
Prepare the tablets as in Example 1.

EXAMPLE 6

Tablets

Formula

| No. | Ingredient | mg/tablet |
|---|---|---|
| 1 | ACE inhibitor | 50 |
| 2 | Lactose | 122 |
| 3 | Corn Starch, Food Grade, as a 10% past in Purified Water | 30 |
| 4 | Corn Starch, Food Grade | 45 |
| 5 | Magnesium Stearate | 3 |
| | Approximate Tablet Weight | 250 |

Method of Manufacturing
Prepare the tablets as in Example 1.

EXAMPLE 7

Tablets

Formula

| No. | Ingredient | mg/tablet | mg/tablet |
|---|---|---|---|
| 1 | Drug | 50 | 400 |
| 2 | Atrial Peptide | 0.1 | 1 |
| 3 | Lactose | 121.9 | 212 |
| 4 | Corn Starch, Food Grade as a 10% Paste in Purified Water | 30 | 40 |
| 5 | Corn Starch, Food Grade | 45 | 40 |
| 6 | Magnesium Stearate | 3 | 7 |
| | | 250 | 700 |

Method of Manufacturing
Prepare the tablets as in Example 1.

EXAMPLE 8

Tablets

Formula

| No. | Ingredient | mg/tablet | mg/tablet |
|---|---|---|---|
| 1 | Atrial Peptide | 0.1 | 1 |
| 2 | Lactose | 61 | 121 |
| 3 | Corn Starch, as a 10% Paste in Purified Water | 15 | 30 |
| 4 | Corn Starch | 22.4 | 45 |
| 5 | Magnesium Stearate | 1.5 | 3 |
| | | 100 | 200 |

Method of Manufacture
Prepare tablets as in Example 1.

Following are descriptions of preparations of typical starting materials and examples of procedures for preparing compounds of formula I.

PREPARATION 1

S-ALLYL-(R)-CYSTEINAMIDE HYDROCHLORIDE
Step 1 : N-t-Butyloxycarbonyl-S-allyl-(R)-cysteine : Treat S-allyl-(R)-cysteine (1.42g) in THF (20 ml) and MeOH (5 ml) with di-t-butyl dicarbonate (2.1 g) and triethylamine (2.5 ml) and stir the resulting mixture at room temperature for 20 hr. Concentrate the mixture in vacuo, dilute with water, and extract with hexane. Acidify the aqueous solution with $KHSO_4$ solution and extract with EtOAc. Concentrate the dried ($MgSO_4$) EtOAc solution in vacuo to give the title compound, a clear oil.

In a similar manner, using the appropriate amino acid, prepare :
N-t-Butyloxycarbonyl-S-methylthiometyl-(R)-cysteine, a white solid, m.p. 79-80°C, $[\alpha]_D^{26} = -33.0°$ (MeOH).

Step 2 : N-t-Butyloxycarbonyl-S-allyl-(R)-cysteinamide : React N-t-butyloxycarbonyl-S-allyl-R-cysteine (2.02 g) with triethylamine (2.5 ml) in THF (25 ml). Cool the mixture to 0-5°C. Add ethyl chloroformate (1.4 ml) in THF (5 ml) dropwise over 5 min. and stir the reaction mixture for 15 min. Add ammonium hydroxide (29%, 0.8 ml) in THF (5 ml) dropwise. Allow the reaction mixture to warm to room temperature and stir for 18 hr. Filter the reaction mixture and concentrate the filtrate in vacuo. Dissolve the resultant residue in EtOAc and extract with $H_2O$ and brine. Concentrate the dried ($MgSO_4$) EtOAc solution in vacuo to give a white solid, m.p. 105-108°C, $[\alpha]_D^{26} = -13.9°$ (MeOH).

In a similar manner, prepare N-t-butyloxycarbonyl-S-methylthiomethyl-(R)-cysteinamide, a white solid, m.p. 108-9°C, $[\alpha]_D^{26} = -21.6°$ (MeOH).

Step 3 : S-Allyl-(R)-cysteinamide hydrochloride : Treat the product of Step 1 (1.52 g) with 9% HCl in dioxane (25 ml) at 0° and keep the resulting mixture at 0° for 20 hr. Concentrate the reaction mixture in vacuo. Triturate with $Et_2O$ and dry in vacuo to give the title compound, a tan solid, m.p. 163-7°C, $[\alpha]_D^{26} = 0.0°$ (MeOH).

In a similar manner, prepare S-methylthiomethyl-(R)-cysteinamide hydrochloride, a tan solid, m.p. 166°C, $[\alpha]_D^{26} = -3.6°$ (MeOH).

## EXAMPLE 1

### N-[3-BENZOYLTHIO-2(S)-BENZYLPROPIONYL]-S-ALLYL-(R)-CYSTEINAMIDE

Add 3-benzoylthio-2(S)-benzylpropionic acid (0.92 g) to S-allyl-(R)-cysteinamide hydrochloride (0.6 g), 1-(3-dimethylaminopropyl)-3-ethylcarbodiimide hydrochloride (DEC) (0.64 g), 1-hydroxybenzotriazole (HOBT) (0.47 g) and N-methylmorpholine (NMM) (0.7 ml) in dimethylformamide (DMF) (5 ml) and stir the resulting mixture at room temperature for 20 hours. Concentrate the reaction mixture in vacuo and partition the residue between ethyl acetate and water. Concentrate the dried ($MgSO_4$) ethyl acetate solution in vacuo. Chromatograph the resultant residue on flash silica gel (200 ml) using ethyl acetate : hexane (7 : 13) (2L), then 1 : 1 as eluent to give the title compound, a white solid, m.p. 152-6°C, $[\alpha]_D^{26} = -88.0°$ (MeOH).

In a similar manner, substitute S-methylthiomethyl-(R)-cysteinamide for the allyl compound to obtain N-[3-benzoylthiomethyl-2(S)-benzylpropionyl]-S-methylthiomethyl-(R)-cysteinamide, a white solid, m.p. 146-50°C, $[\alpha]_D^{26} = -101.1°$ (MeOH).

In a similar manner, substitute 3-acetylthio-2(S)-benzylpropionic acid or the corresponding 2(R) compound for the 3-benzoylthio compound to obtain :

N-[3-acetylthio-2(S)-benzylpropionyl]-S-allyl-(R)-cysteinamide, a white solid, m.p. 116-20°C, $[\alpha]_D^{26} = -61.6°$ (MeOH) ;

N-[3-acetylthio-2(R)-benzylpropionyl]-S-allyl-(R)-cysteinamide, a tan solid, m.p. 95-9°C, $[\alpha]_D^{26} = -1.6°$ (MeOH) ;

N-[3-acetylthio-2(S)-benzylpropionyl]-S-methylthiomethyl-(R)-cysteinamide, a white solid, m.p. 106-8°C, $[\alpha]_D^{26} = -59.1°$ (MeOH) ; and

N-[3-acetylthio-2(R)-benzylpropionyl]-S-methylthiomethyl-(R)-cysteinamide, a light orange solid, m.p. 84-5°C, $[\alpha]_D^{26} = -8.6°$ (MeOH).

Again in a similar manner, substitute 3-acetylthio-2(S)-benzylpropionic acid or the corresponding 2(R) compound for the 3-benzoylthio compound and substitute S-methylthiomethyl-(R)-cysteine ethyl ester for the corresponding amide to obtain :

N-[3-acetylthio-2(S)-benzylpropionyl]-S-methylthiomethyl-(R)-cysteine ethyl ester, a white solid, m.p. 52-3°C, $[\alpha]_D^{26} = -76.4°$ (MeOH) ; and

N-[3-acetylthio-2(R)-benzylpropionyl]-S-methylthiomethyl-(R)-cysteine ethyl ester, a white solid, m.p. 56-7°C, $[\alpha]_D^{26} = -7.7°$ (MeOH).

EXAMPLE 2

N-[2(S)-ACETYLTHIOMETHYL-3-PHENYLPROPIONYL)-S-ALLYL-(R)-CYSTEINE 2-(2-CHLOROETHOXY)ETHYL ESTER

Step 1 : Combine N-(t-butoxycarbonyl)-S-allyl-(R)-cysteine (5.0 g), 2-(2-chloroethoxy)ethanol (2.38 g) and 4-dimethylaminopyridine (20 mg) in THF (25 ml). Add DEC (4.02 g). Add DMF (10 ml), stir overnight, concentrate, and partition between EtOAc and 1N HCl. Dry ($MgSO_4$) and concentrate to obtain the 2-(2-chloroethoxy)ethyl ester as an amber oil, $[\alpha]_D^{26} = -22.4°$ (MeOH).

Step 2 : Treat the ester of Step 1 with 9% HCl/dioxane (10 ml). Store at 0° overnight, concentrate and triturate the residue with $Et_2O$ to obtain the hydrochloride as a tan solid, m.p. 84-6°C, $[\alpha]_D^{26} = -0.6°$ (MeOH).

Step 3 : In a manner similar to that described in Example 1, step 1, condense the hydrochloride of Step 2 (1.8 g) with 2(S)-acetylthiomethyl-3-phenylpropionic acid (1.41 g) and chromatograph to obtain the title compound as a colorless oil, $[\alpha]_D^{26} = -52.8°$ (MeOH).

In a similar manner, substituting 2(R)-acetylthiomethyl-3-phenylpropionyl chloride in Step 3, obtain :

N-[2(R)-acetylthiomethyl-3-phenylpropionyl)-S-allyl-(R)-cysteine 2(2-chloroethoxy)ethyl ester, a colorless oil, $[\alpha]_D^{26} = +9.8°$ (MeOH).

EXAMPLE 3

N-[2(S)-BENZOYLTHIO-3-PHENYLPROPIONYL)-S-ALLYL-(R)-CYSTEINE 2-(2-CHLOROETHOXY)ETHYL ESTER

Combine the product of Example 2, Step 2 (0.704 g) with 2(S)-benzoylthiomethyl-3-phenylpropionic acid (0.7 g), HOBT (0.36 g) and NMM (0.49 g) in DMF (5 ml). Add DEC (0.49 g) and stir 18 hours. Concentrate and partition between EtOAc and $H_2O$. Wash with IN HCl, brine, then dry and concentrate. Chromatograph the resultant residue on flash silica gel (200 ml) (EtOAc : hexane 1 : 4 as eluant) to obtain the title compound, a white solid, m.p. 71-4°C, $[\alpha]_D^{26} = -55.2°$ (MeOH).

Using a procedure similar to that described in Steps 1 and 2 of Example 2, prepare N-t-(butoxycarbonyl)-S-methylthiomethyl-(R)-cysteine 2-(2-chloroethoxy)ethyl ester hydrochloride, and then following a procedure similar to that immediately above, prepare :

N-[2(S)-benzoylthio-3-phenylpropionyl)-S-methylthiomethyl-(R)-cysteine 2-(2-chloroethoxy)ethyl ester, a white solid, m.p. 70-1°C, $[\alpha]_D^{26} = -63.5°$ (MeOH).

## Claims

### Claims for the Contracting States : AT, BE, CH, DE, FR, GB, GR, IT, LI, LU, NL, SE

1. A compound having the structural formula

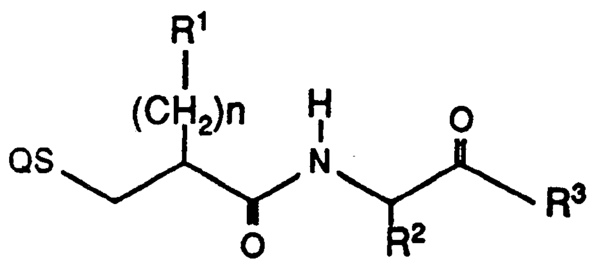

wherein $R^1$ is $Y\text{-}C_6H_4-$, $Y\text{-}C_6H_4S-$, $Y\text{-}C_6H_4O-$,

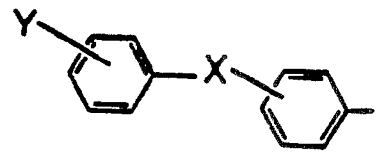

α-naphthyl, ß-naphthyl, furyl, thienyl, benzofuryl, benzothienyl, $H_2N(CH_2)_m-$, diphenylmethyl,

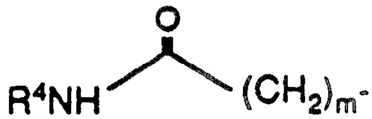

$R^2$ is $R^{14}(CH_2)_kS(O)_{0-2}(CH_2)_q$- or $R^6OCO(CH_2)_q$- ;
$R^3$ is $-OR^7$, $-NR^7R^8$,

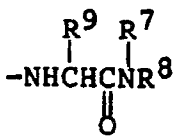, 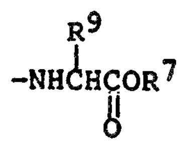 or 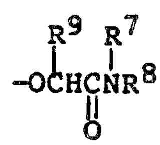 ;

$R^4$ is hydrogen, alkyl or $Y^1$-$C_6H_4-$ ;
$R^{14}$ is mono-unsaturated lower alkyl, hydroxy, alkoxy or alkylthio, provided that when $R^{14}$ is hydroxy or alkoxy, k is 2 or 3 and when $R^{14}$ is mono-unsaturated alkyl or alkylthio, k is 1, 2 or 3 ;
$R^6$ is dihydroxyalkyl, dialkoxyalkyl, alkoxyalkoxyalkyl, haloalkyl, (haloalkoxy)alkyl or alkyl substituted with a 5-6 membered saturated ring comprising 1-2 oxygen atoms as ring members wherein the ring carbon atoms may be substituted with 0-2 alkyl substituents ;
$R^7$ and $R^8$ are independently $R^6$, H, alkyl, hydroxyalkyl, alkoxyalkyl, aminoalkyl, alkylaminoalkyl, dialkylaminoalkyl or arylalkyl, $R^7$ and $R^8$ together with the nitrogen to which they are attached complete a 5-7 membered ring, wherein one of the 4-6 ring members comprising $R^7$ and $R^8$ may be a nitrogen atom, an alkyl-substituted nitrogen atom or an oxygen atom, and wherein the ring may be substituted on the ring carbon atoms with substituents chosen from alkyl and hydroxy groups ;
$R^9$ is hydrogen, alkyl, carboxyalkyl, mercaptoalkyl, alkylthioalkyl, aminoalkyl, hydroxyalkyl, phenylalkyl, hydroxyphenylalkyl, guanidinoalkyl, imidazolylalkyl, indolylalkyl, or carbamoylalkyl ;
n is 0-2 ;
m and k are independently 0-3 ;
q is 1-4 ;
X is a bond, –O–, –S–, or $-CH_2-$ ;
Q is hydrogen or $R^{10}CO-$ ;
$R^{10}$ is alkyl, hydroxyalkyl, alkoxyalkyl, dialkylaminoalkyl, $Y^2$-$C_6H_4$-alkyl, alkoxy, $Y^2$-$C_6H_4-$, naphthyl, furyl, thienyl or pyridyl ;
Y, $Y^1$ and $Y^2$ independently represent one or more substituents selected from H, alkyl, cycloalkyl, alkoxy, OH, F, Cl, Br, CN, $-CH_2NH_2$, $-CO_2H$, $-CO_2$alkyl, $-CONH_2$ and phenyl ;
and the pharmaceutically acceptable addition salts thereof.

2. A compound according to claim 1 wherein $R^2$ is $R^{14}(CH_2)_kS(O)_{0-2}(CH_2)_q-$.

3. A compound according to claim 2 wherein $R^{14}$ is mono-unsaturated lower alkyl or alkylthio.

4. A compound according to any one of claims 1 to 3 wherein $R^1$ is Y-$C_6H_4-$.

5. A compound according to any one of claims 1 to 4 wherein Q is hydrogen or $R^{10}CO-$ wherein $R^{10}$ is alkyl or phenyl.

6. A compound according to any one of claims 1 to 5 wherein $R^3$ is $-OR^7$ or $-NR^7R^8$.

7. A compound according to claim 1 which is
N-[3-benzoylthio-2(S)-benzylpropionyl]-S-allyl-(R)-cysteinemide ;
N-[3-benzoylthiomethyl-2(S)-benzylpropionyl]-S-methylthiomethyl-(R)-cysteinamide ;
N-[3-acetylthio-2(S)-benzylpropionyl]-S-allyl-(R)-cysteinamide ;
N-[3-acetylthio-2(R)-benzylpropionyl]-S-allyl-(R)-cysteinamide ;
N-[3-acetylthio-2(S)-benzylpropionyl]-S-methylthiomethyl-(R)-cysteinamide ;
N-[3-acetylthio-2(R)-benzylpropionyl]-S-methylthiomethyl-(R)-cysteinamide ;
N-[3-acetylthio-2(S)-benzylpropionyl]-S-methylthiomethyl-(R)-cysteine ethyl ester ;
N-[3-acetylthio-2(R)-benzylpropionyl]-S-methylthiomethyl-(R)-cysteine ethyl ester ;
N-[2(S)-acetylthiomethyl-3-phenylpropionyl)-S-allyl-(R)-cysteine 2-(2-chloroethoxy)ethyl ester ;
N-[2(R)-acetylthiomethyl-3-phenylpropionyl)-S-allyl-(R)-cysteine 2-(2-chloroethoxy)ethyl ester ;
N-[2(S)-benzoylthio-3-phenylpropionyl)-S-allyl-(R)-cysteine 2-(2-chloroethoxy)ethyl ester ;
N-[2(s)-benzoylthio-3-phenylpropionyl)-S-methylthiomethyl-(R)-cysteine 2-(2-chloroethoxy)ethyl ester ; and
ß-benzyl N-[2(R,S)-mercaptomethyl-3-phenylpropionyl]-(S)-aspartate.

8. A pharmaceutical composition comprising as active ingredient a compound as claimed in any one of claims 1 to 7.

9. The use of a compound as claimed in any of claims 1 to 7 for the preparation of a pharmaceutical composition for the treatment of hypertension and/or congestive heart failure.

10. Process for the preparation of a compound of formula I as defined in claim 1, wherein the compound is prepared by an appropriate process selected from the following processes A and B, and wherein Q, $R^1$, $R^2$, $R^3$ and n are defined in claim 1, including suitable protection :

Process A : condensation of a 3-thiopropionic acid of formula IV, or a reactive derivative thereof, with an amine of formula V

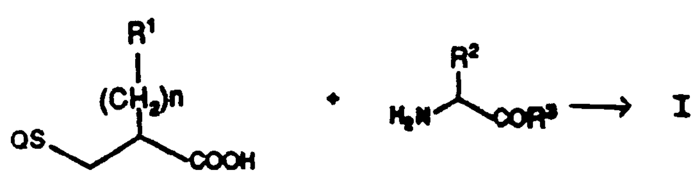

IV V

Process B : for compounds of formula I wherein $R^3$ is $-NR^7R^8$, condensation of a (3-thiopropionyl)amino acid of formula VI, or a reactive derivative thereof, with an amine of formula VII

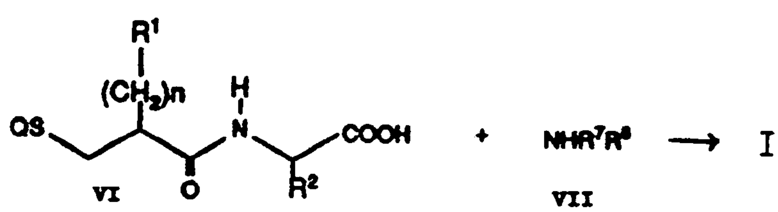

wherein both Process A and Process B are followed by isolating the preferred isomer if desired and removal of the protecting groups, if necessary, to yield the desired products, and if desired, preparing a salt thereof.

**Claims for Contracting State : ES**

1. A process for the production of a compound having the structural formula

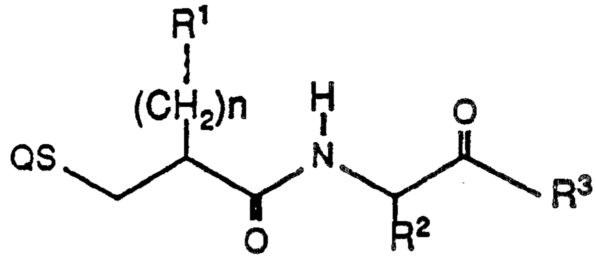

wherein $R^1$ is Y-$C_6H_4$–, Y-$C_6H_4S$–, Y-$C_6H_4O$–,

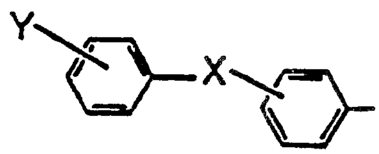

α-naphthyl, ß-naphthyl, furyl, thienyl, benzofuryl, benzothienyl, $H_2N(CH_2)_m$–, diphenylmethyl,

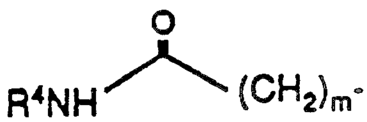

$R^2$ is $R^{14}(CH_2)_kS(O)_{0-2}(CH_2)_q$– or $R^6OCO(CH_2)_q$– ;
$R^3$ is $-OR^7$, $-NR^7R^8$,

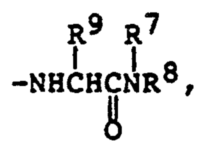, 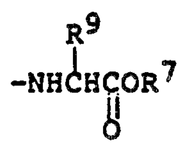 or 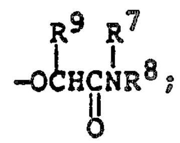;

$R^4$ is hydrogen, alkyl or $Y^1$-$C_6H_4$– ; $R^{14}$ is mono-unsaturated lower alkyl, hydroxy, alkoxy or alkylthio, provided that when $R^{14}$ is hydroxy or alkoxy, k is 2 or 3 and when $R^{14}$ is mono-unsaturated alkyl or alkylthio, k is 1, 2 or 3 ;
$R^6$ is dihydroxyalkyl, dialkoxyalkyl, alkoxyalkoxyalkyl, haloalkyl, (haloalkoxy)alkyl or alkyl substituted with a 5-6 membered saturated ring comprising 1-2 oxygen atoms as ring members wherein the ring carbon atoms may be substituted with 0-2 alkyl substituents ;
$R^7$ and $R^8$ are independently $R^6$, H, alkyl, hydroxyalkyl, alkoxyalkyl, aminoalkyl, alkylaminoalkyl, dialkylaminoalkyl or arylalkyl, $R^7$ and $R^8$ together with the nitrogen to which they are attached complete a 5-7 membered ring, wherein one of the 4-6 ring members comprising $R^7$ and $R^8$ may be a nitrogen atom, an alkyl-substituted nitrogen atom or an oxygen atom, and wherein the ring may be substituted on the ring carbon atoms with substituents chosen from alkyl and hydroxy groups ;
$R^9$ is hydrogen, alkyl, carboxyalkyl, mercaptoalkyl, alkylthioalkyl, aminoalkyl, hydroxyalkyl, phenylalkyl, hydroxyphenylalkyl, guanidinoalkyl, imidazolylalkyl, indolylalkyl, or carbamoylalkyl ;
n is 0-2 ; m and k are independently 0-3 ;
q is 1-4 ;
X is a bond, –O–, –S–, or $-CH_2-$
Q is hydrogen or $R^{10}CO$– ;
$R^{10}$ is alkyl, hydroxyalkyl, alkoxyalkyl, dialkylaminoalkyl, $Y^2$-$C_6H_4$-alkyl, alkoxy, $Y^2$-$C_6H_4$–, naphthyl, furyl, thienyl or pyridyl ; Y, $Y^1$ and $Y^2$ independently represent one or more substituents selected from H, alkyl, cycloalkyl, alkoxy, OH, F, Cl, Br, CN, $-CH_2NH_2$, $-CO_2H$, $-CO_2$alkyl, $-CONH_2$ and phenyl ;
and the pharmaceutically acceptable addition salts thereof,

wherein the compound is prepared by an appropriate process selected from the following processes A and B, and wherein Q, $R^1$, $R^2$, $R^3$ and n are defined in claim 1, including suitable protection :

Process A : condensation of a 3-thiopropionic acid of formula IV, or a reactive derivative thereof, with an amine of formula V

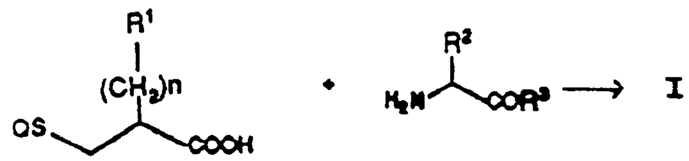

IV V

Process B : for compounds of formula I wherein $R^3$ is $-NR^7R^8$, condensation of a (3-thiopropionyl)amino acid of formula VI, or a reactive derivative thereof, with an amine of formula VII

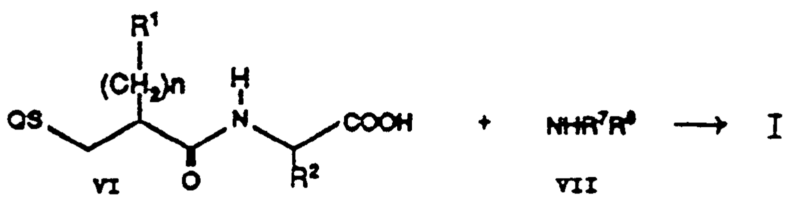

VI VII

wherein both Process A and Process B are followed by isolating the preferred isomer if desired and removal of the protecting groups, if necessary, to yield the desired products, and if desired, preparing a salt thereof.

2. Process according to claim 1 for the production of a compound wherein $R^2$ is $R^{14}(CH_2)_k(S(O)_{0-2}(CH_2)_q-$.

3. Process according to claim 2 for the production of a compound wherein $R^{14}$is mono-unsaturated lower alkyl or alkylthio.

4. Process according to claims 1 to 3 for the production of a compound wherein $R^1$ is $Y-C_6H_4-$.

5. Process according to claims 1 to 4 for the production of a compound wherein Q is hydrogen or $R^{10}CO-$ wherein $R^{10}$is alkyl or phenyl.

6. Process according to claims 1 to 5 for the production of a compound wherein $R^3$ is $-OR^7$ or $-NR^7R^8$.

7. Process according to claim 1 for the production of a compound which is
N-[3-benzoylthio-2(S)-benzylpropionyl]-S-allyl-(R)-cysteinemide ;
N-[3-benzoylthiomethyl-2(S)-benzylpropionyl]-S-methylthiomethyl-(R)-cysteinamide ;
N-[3-acetylthio-2(S)-benzylpropionyl]-S-allyl-(R)-cysteinamide ;
N-[3-acetylthio-2(R)-benzylpropionyl]-S-alllyl-(R)-cystelnamide ;
N-[3-acetylthio-2(S)-benzylpropionyl]-S-methylthiomethyl-(R)-cysteinamide ;
N-[3-acetylthio-2(R)-benzylpropionyl]-S-methylthiomethyl-(R)-cysteinamide ;
N-[3-acetylthio-2(S)-benzylpropionyl]-S-methylthiomethyl-(R)-cysteine ethyl ester ;
N-[3-acetylthio-2(R)-benzylpropionyl]-S-methylthiomethyl-(R)-cysteine ethyl ester ;
N-(2(S)-acetylthiomethyl-3-phenylpropionyl)-S-allyl-(R)-cysteine 2-(2-chloroethoxy)ethyl ester ;
N-[2(R)-acetylthiomethyl-3-phenylpropionyl)-S-allyl-(R)-cysteine 2(2-chloroethoxy)ethyl ester ;
N-(2(S)-benzoylthio-3-phenylpropionyl)-S-allyl-(R)-cysteine 2-(2-chloroethoxy)ethyl ester ;
N-(2(s)-benzoylthio-3-phenylpropionyl)-S-methylthiomethyl-(R)-cysteine 2-(2-chloroethoxy)ethyl ester ;
and
ß-benzyl N-[2(R,S)-mercaptomethyl-3-phenylpropionyl]-(S)-aspartate.

8. Process for the production of a pharmaceutical composition comprising as active ingredient a compound produced according to a process as claimed in any one of claims 1 to 7, wherein the active ingredient is mixed with a pharmaceutically acceptable carrier.

9. The use of a compound produced according to a process as claimed in any of claims 1 to 7 for the preparation of a pharmaceutical composition for the treatment of hypertension and/or congestive heart failure.

## Ansprüche

**Patentansprüche für die Vestragsstaaten : AT, BE, CH, DE, FR, GB, GR, IT, LI, LU, NL, SE**

1. Verbindung der Strukturformel

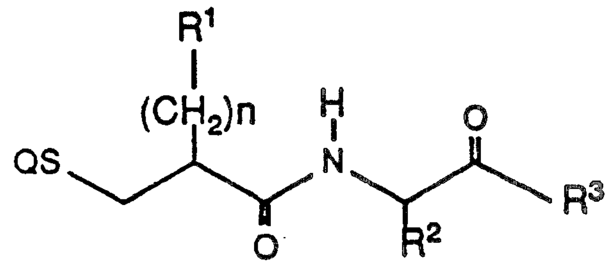

in der
$R^1$ Y-$C_6H_4$–, Y-$C_6H_4S$–, Y-$C_6H_4O$–,

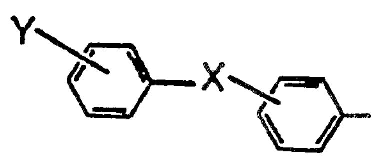

α-Naphthyl, ß-Naphthyl, Furyl, Thienyl, Benzofuryl, Benzothienyl, $H_2N(CH_2)_m$–, Diphenylmethyl oder

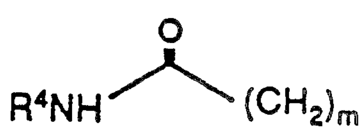

ist;
$R^2$ $R^{14}(CH_2)_kS(O)_{0-2}(CH_2)_q$– oder $R^6OCO(CH_2)_q$– ist;
$R^3$ $-OR^7$, $-NR^7R^8$,

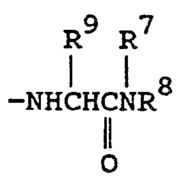, 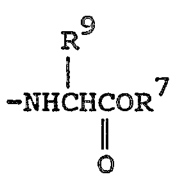, 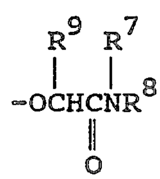

ist,
$R^4$ Wasserstoff, Alkyl oder $Y^1$-$C_6H_4$ ist;
$R^{14}$ einfach ungesättigtes Niederalkyl, Hydroxy, Alkoxy oder Alkylthio ist, mit der Maßgabe, daß dann, wenn $R^{14}$ Hydroxy oder Alkoxy ist, k 2 oder 3 ist, und wenn $R^{14}$ einfach ungesättigtes Niederalkyl oder Alkylthio ist, k 1, 2 oder 3 ist;
$R^6$ Dihydroxyalkyl, Dialkoxyalkyl, Alkoxyalkoxyalkyl, Halogenoalkyl, (Halogenoalkoxy)alkyl oder ein Alkyl ist, das mit einem 5- oder 6-gliedrigen gesättigten Ring, der 1 bis 2 Sauerstoff-Atome als Ring-Glieder umfaßt, substituiert ist, worin die Ring-Kohlenstoff-Atome mit 0 bis 2 Alkyl-Substituenten substituiert sein können;
$R^7$ und $R^8$ unabhängig voneinander $R^6$, H, Alkyl, Hydroxyalkyl, Alkoxyalkyl, Aminoalkyl, Alkylaminoalkyl, Dialkylaminoalkyl oder Arylalkyl sind, $R^7$ und $R^8$, zusammen mit dem Stickstoff, an den sie gebunden sind, einen 5- bis 7-gliedrigen Ring vervollständigen, worin eines der $R^7$ und $R^8$ umfassenden 4 bis 6 Ring-Glieder ein Stickstoff-Atom, ein alkyl-substituiertes Stickstoff-Atom oder ein Sauerstoff-Atom ist, und worin der Ring an den Ring-Kohlenstoff-Atomen mit Substituenten substituiert sein kann, die aus Alkyl- und Hydroxy-Gruppen ausgewählt sind;

$R^9$ Wasserstoff, Alkyl, Carboxyalkyl, Mercaptoalkyl, Alkylthioalkyl, Aminoalkyl, Hydroxyalkyl, Phenylalkyl, Hydroxyphenylalkyl, Guanidinoalkyl, Imidazolylalkyl, Indolylalkyl oder Carbamoylalkyl ist;
n 0 bis 2 ist;
m und k unabhängig voneinander 0 bis 3 sind;
q 1 bis 4 ist;
X eine Bindung, $-O-$, $-S-$ oder $-CH_2-$ ist;
Q Wasserstoff oder $R^{10}CO-$ ist;
$R^{10}$ Alkyl, Hydroxyalkyl, Alkoxyalkyl, Dialkylaminoalkyl, $Y^2$-$C_6H_4$-alkyl, Alkoxy, $Y^2$-$C_6H_4-$, Naphthyl, Furyl, Thienyl oder Pyridyl ist;
Y, $Y^1$ oder $Y^2$ unabhängig voneinander einen oder mehrere Substituenten darstellen, die aus H, Alkyl, Cycloalkyl, Alkoxy, OH, F, Cl, Br, CN, $-CH_2NH_2$, $-CO_2H$, $-CO_2$-alkyl, $-CONH_2$ und Phenyl ausgewählt sind;
und deren pharmazeutisch annehmbare Säureadditionssalze.

2. Verbindung nach Anspruch 1, worin $R^2$ $R^{14}(CH_2)_kS(O)_{0-2}(CH_2)_q-$ ist.

3. Verbindung nach Anspruch 2, worin $R^{14}$ einfach ungesättigtes Niederalkyl oder Alkylthio ist.

4. Verbindung nach irgendeinem der Ansprüche 1 bis 3, worin $R^1$ Y-$C_6H_4-$ ist.

5. Verbindung nach irgendeinem der Ansprüche 1 bis 4, worin Q Wasserstoff oder $R^{10}CO-$ ist, worin $R^{10}$ Alkyl oder Phenyl ist.

6. Verbindung nach irgendeinem der Ansprüche 1 bis 5, worin $R^3$ $-OR^7$ oder $-NR^7R^8$ ist.

7. Verbindung nach Anspruch 1, die
N-[3-Benzoylthio-2(S)-benzylpropionyl]-S-allyl-(R)-cysteinamid;
N-[3-Benzoylthiomethyl-2(S)-benzylpropionyl]-S-methyl-thiomethyl-(R)-cysteinamid;
N-[3-Acetylthio-2(S)-benzylpropionyl]-S-allyl-(R)-cysteinamid;,
N-[3-Acetylthio-2(R)-benzylpropionyl]-S-allyl-(R)-cysteinamid;
N-[3-Acetylthio-2(S)-benzylpropionyl]-S-methylthiomethyl-(R)-cysteinamid;
N-[3-Acetylthio-2(R)-benzylpropionyl]-S-methylthiomethyl-(R)-cysteinamid;
N-[3-Acetylthio-2(S)-benzylpropionyl]-S-methylthiomethyl-(R)-cysteinethylester;
N-[3-Acetylthio-2(R)-benzylpropionyl]-S-methylthiomethyl-(R)-cysteinethylester;
N-[2(S)-Acetylthiomethyl-3-phenylpropionyl]-S-allyl-(R)-cystein-2-(2-chloroethoxy)ethylester;
N-[2(R)-Acetylthiomethyl-3-phenylpropionyl]-S-allyl-(R)-cystein-2-(2-chloroethoxy)ethylester;
N-[2(S)-Benzoylthio-3-phenylpropionyl]-S-allyl-(R)-cystein-2-(2-chloroethoxy)ethylester;
N-[2(S)-Benzoylthio-3-phenylpropionyl]-S-methylthiomethyl-(R)-cystein-2-(2-chloroethoxy)ethylester;
und
ß-Benzyl-N-[2(R,S)-mercaptomethyl-3-phenylpropionyl]-(S)-aspartat ist.

8. Pharmazeutische Zusammensetzung, umfassend als Wirkstoff eine Verbindung nach irgendeinem der Ansprüche 1 bis 7.

9. Verwendung einer Verbindung nach irgendeinem der Ansprüche 1 bis 7 zur Herstellung einer pharmazeutischen Zusammensetzung zur Behandlung von Hypertonie und/oder kongestiver Herz-Insuffizienz.

10. Verfahren zur Herstellung einer Verbindung der Formel I, wie sie in Anspruch 1 definiert ist, wobei die Verbindung mittels eines geeigneten, aus den nachfolgenden Verfahren A und B ausgewählten Verfahrens hergestellt wird, und worin Q, $R^1$, $R^2$, $R^3$ und n die in Anspruch 1 angegebenen Bedeutungen haben, einschließlich geeigneten Schutzes:

Verfahren A:

Kondensation einer 3-Thiopropionsäure der Formel IV oder eines reaktionsfähigen Derivats derselben mit einem Amin der Formel V

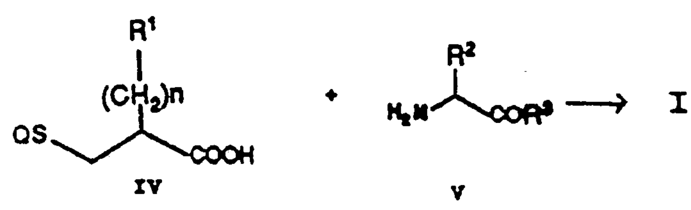

Verfahren B:

für Verbindungen der Formel I, in der $R^3$ $-NR^7R^8$ ist, Kondensation einer (3-Thiopropionyl)aminosäure der Formel VI oder eines reaktionsfähigen Derivats derselben mit einem Amin der Formel VII

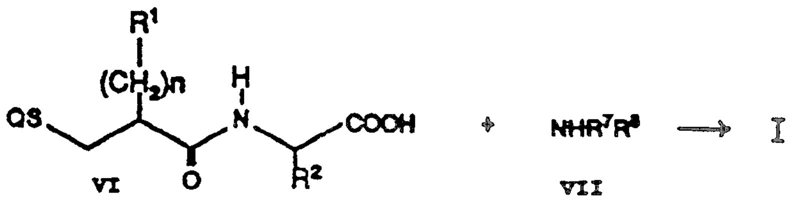

VI VII

wobei sowohl im Anschluß an Verfahren A als auch im Anschluß an Verfahren B gewünschtenfalls das bevorzugte Isomer isoliert wird und erforderlichenfalls die Schutzgruppen entfernt werden, um die gewünschten Produkte zu erhalten, und,
sofern gewünscht, ein Salz derselben hergestellt wird.

## Patentansprüche für den Vertragsstaat : ES

1. Verfahren zur Herstellung einer Verbindung der Strukturformel

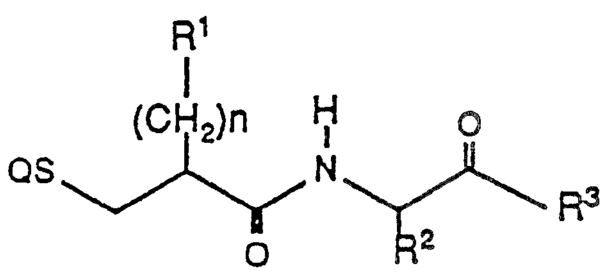

in der
$R^1$ $Y$-$C_6H_4$–, $Y$-$C_6H_4S$–, $Y$-$C_6H_4O$–,

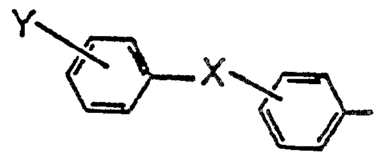

α-Naphthyl, ß-Naphthyl, Furyl, Thienyl, Benzofuryl, Benzothienyl, $H_2N(CH_2)_m$–, Diphenylmethyl oder

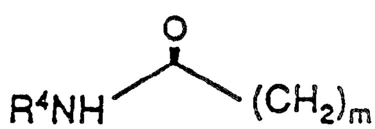

ist ;
$R^2$ $R^{14}(CH_2)_kS(O)_{0-2}(CH_2)_q$– oder $R^6OCO(CH_2)_q$– ist ;
$R^3$ $-OR^7$, $-NR^7R^8$,

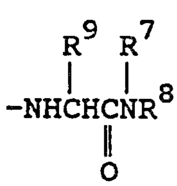 , 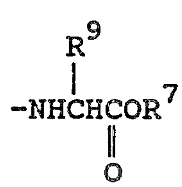 , 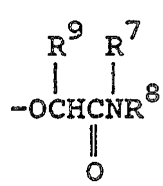

ist ;
$R^4$ Wasserstoff, Alkyl oder $Y^1$-$C_6H_4$ ist ;
$R^{14}$ einfach ungesättigtes Niederalkyl, Hydroxy, Alkoxy oder Alkylthio ist, mit der Maßgabe, daß dann, wenn $R^{14}$ Hydroxy oder Alkoxy ist, k 2 oder 3 ist, und wenn $R^{14}$ einfach ungesättigtes Niederalkyl oder Alkylthio ist, k 1, 2 oder 3 ist ;
$R^6$ Dihydroxyalkyl, Dialkoxyalkyl, Alkoxyalkoxyalkyl, Halogenoalkyl, (Halogenoalkoxy)alkyl oder ein Alkyl ist, das mit einem 5- oder 6-gliedrigen gesättigten Ring, der 1 bis 2 Sauerstoff-Atome als Ring-Glieder umfaßt, substituiert ist, worin die Ring-Kohlenstoff-Atome mit 0 bis 2 Alkyl-Substituenten substituiert sein können ;
$R^7$ und $R^8$ unabhängig voneinander $R^6$, H, Alkyl, Hydroxyalkyl, Alkoxyalkyl, Aminoalkyl, Alkylaminoalkyl, Dialkylaminoalkyl oder Arylalkyl sind, $R^7$ und $R^8$, zusammen mit dem Stickstoff, an den sie gebunden sind, einen 5- bis 7-gliedrigen Ring vervollständigen, worin eines der $R^7$ und $R^8$ umfassenden 4 bis 6 Ring-Glieder ein Stickstoff-Atom, ein alkyl-substituiertes Stickstoff-Atom oder ein Sauerstoff-Atom ist, und worin der Ring an den Ring-Kohlenstoff-Atomen mit Substituenten substituiert sein kann, die aus Alkyl- und Hydroxy-Gruppen ausgewählt sind ;
$R^9$ Wasserstoff, Alkyl, Carboxyalkyl, Mercaptoalkyl, Alkylthioalkyl, Aminoalkyl, Hydroxyalkyl, Phenylalkyl, Hydroxyphenylalkyl, Guanidinoalkyl, Imidazolylalkyl, Indolylalkyl oder Carbamoylalkyl ist ;
n 0 bis 2 ist ;
m und k unabhängig voneinander 0 bis 3 sind ;
q 1 bis 4 ist ;
X eine Bindung, –O–, –S– oder $-CH_2-$ ist ;
Q Wasserstoff oder $R^{10}CO$– ist ;
$R^{10}$ Alkyl, Hydroxyalkyl, Alkoxyalkyl, Dialkylaminoalkyl, $Y^2$-$C_6H_4$-alkyl, Alkoxy, $Y^2$-$C_6H_4$–, Naphthyl, Furyl, Thienyl oder Pyridyl ist ;
Y, $Y^1$ oder $Y^2$ unabhängig voneinander einen oder mehrere Substituenten darstellen, die aus H, Alkyl, Cycloalkyl, Alkoxy, OH, F, Cl, Br, CN, $-CH_2\,NH_2$, $-CO_2\,H$, $-CO_2$-alkyl, $-CONH_2$ und Phenyl ausgewählt sind ;
und pharmazeutisch annehmbarer Säureadditionssalze derselben,
wobei die Verbindung mittels eines geeigneten, aus den nachfolgenden Verfahren A und B ausgewählten Verfahrens hergestellt wird, und worin Q, $R^1$, $R^2$, $R^3$ und n die in Anspruch 1 angegebenen Bedeutungen haben, einschließlich geeigneten Schutzes :

Verfahren A :
Kondensation einer 3-Thiopropionsäure der Formel IV oder eines reaktionsfähigen Derivats derselben mit einem Amin der Formel V

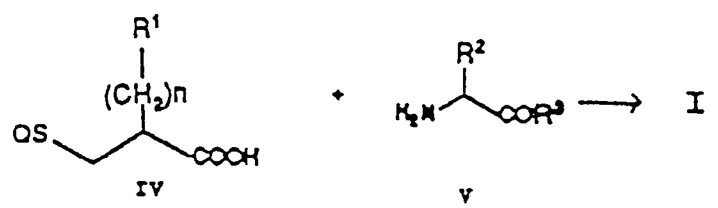

Verfahren B :
für Verbindungen der Formel I, in der $R^3$ $-NR^7R^8$ ist, Kondensation einer (3-Thiopropionyl)aminosäure der Formel VI oder eines reaktionsfähigen Derivats derselben mit einem Amin der Formel VII

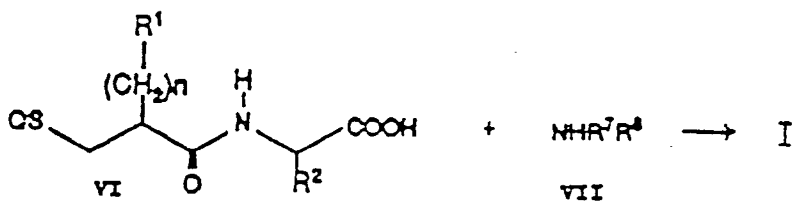

wobei sowohl im Anschluß an Verfahren A als auch im Anschluß an Verfahren B gewünschtenfalls das bevorzugte Isomer isoliert wird und erforderlichenfalls die Schutzgruppen entfernt werden, um die gewünschten Produkte zu erhalten, und, sofern gewünscht, ein Salz derselben hergestellt wird.
2. Verfahren nach Anspruch 1 zur Herstellung einer Verbindung, in der $R^2$ $R^{14}(CH_2)_k\,S(O)_{0-2}\,(CH_2)_q$– ist.

3. Verfahren nach Anspruch 2 zur Herstellung einer Verbindung, in der $R^{14}$ einfach ungesättigtes Niederalkyl oder Alkylthio ist.

4. Verfahren nach irgendeinem der Ansprüche 1 bis 3 zur Herstellung einer Verbindung, in der $R^1$ Y-$C_6H_4$– ist.

5. Verfahren nach irgendeinem der Ansprüche 1 bis 4 zur Herstellung einer Verbindung, in der Q Wasserstoff oder $R^{10}CO$– ist, worin $R^{10}$ Alkyl oder Phenyl ist.

6. Verfahren nach irgendeinem der Ansprüche 1 bis 5 zur Herstellung einer Verbindung, in der $R^3$ –$OR^7$ oder –$NR^7R^8$ ist.

7. Verfahren nach Anspruch 1 zur Herstellung einer Verbindung, die
N-[3-Benzoylthio-2(S)-benzylpropionyl]-S-allyl-(R)-cysteinamid ;
N-[3-Benzoylthiomethyl-2(S)-benzylpropionyl]-S-methyl-thiomethyl-(R)-cysteinamid ;
N-[3-Acetylthio-2(S)-benzylpropionyl]-S-allyl-(R)-cysteinamid ;
N-[3-Acetylthio-2(R)-benzylpropionyl]-S-allyl-(R)-cysteinamid ;
N-[3-Acetylthio-2(S)-benzylpropionyl]-S-methylthiomethyl-(R)-cysteinamid ;
N-[3-Acetylthio-2(R)-benzylpropionyl]-S-methylthiomethyl-(R)-cysteinamid ;
N-[3-Acetylthio-2(S)-benzylpropionyl]-S-methylthiomethyl-(R)-cysteinethylester ;
N-[3-Acetylthio-2(R)-benzylpropionyl]-S-methylthiomethyl-(R)-cysteinethylester ;
N-[2(S)-Acetylthiomethyl-3-phenylpropionyl]-S-allyl-(R)-cystein-2-(2-chloroethoxy)ethylester ;
N-[2(R)-Acetylthiomethyl-3-phenylpropionyl]-S-allyl-(R)-cystein-2-(2-chloroethoxy)ethylester ;
N-[2(S)-Benzoylthio-3-phenylpropionyl]-S-allyl-(R)-cystein-2-(2-chloroethoxy)ethylester ;
N-[2(S)-Benzoylthio-3-phenylpropionyl]-S-methylthiomethyl-(R)-cystein-2-(2-chloroethoxy)ethylester ;
und β-Benzyl-N-[2(R,S)-mercaptomethyl-3-phenylpropionyl]-(S)-aspartat ist.

8. Verfahren zur Herstellung einer pharmazeutischen Zusammensetzung, umfassend als Wirkstoff eine Verbindung nach irgendeinem der Ansprüche 1 bis 7, wobei der Wirkstoff mit einem pharmazeutisch annehmbaren Träger vermischt wird.

9. Verwendung einer Verbindung, die mittels eines Verfahrens hergestellt wird, das in irgendeinem der Ansprüche 1 bis 7 beansprucht wird, zur Herstellung einer pharmazeutischen Zusammensetzung zur Behandlung von Hypertonie und/oder kongestiver Herz-Insuffizienz.

## Revendications

### Revendications pour les Etats Contractants AT, BE, CH, DE, FR, GB, GR, IT, LI, LU, NL, SE

1. Composé ayant la formule de structure

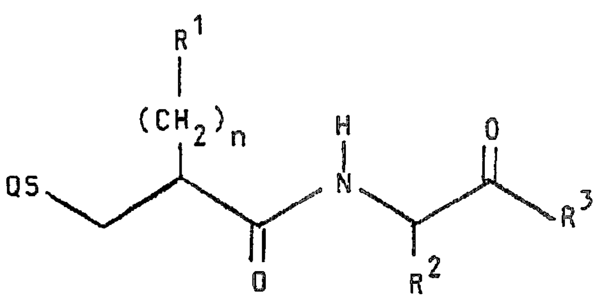

où $R^1$ est Y-$C_6H_4$–, Y-$C_6H_4S$–, Y-$C_6H_4O$–,

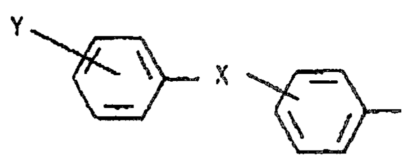

α-naphtyle, β-naphtyle, furyle, thiényle, benzofuryle, benzothiényle, $H_2N(CH_2)_m$–, diphénylméthyle, ou

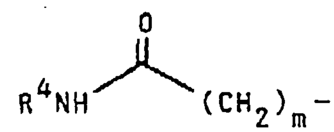

$R^2$ est $R^{14}(CH_2)_kS(O)_{0-2}(CH_2)_q-$ ou $R^6OCO(CH_2)_q-$ ;

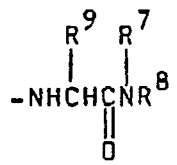, 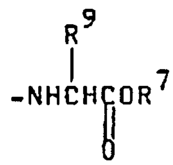 ou 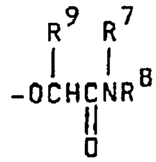

$R^3$ est $-OR^7$ $-NR^7R^8$,

$R^4$ est hydrogène, alkyle ou $Y^1-C_6H_4-$ ;

$R^{14}$ est alkyle inférieur mono-insaturé, hydroxy, alcoxy ou alkylthio à condition que quand $R^{14}$ est hydroxy ou alcoxy, k soit 2 ou 3 et quand $R^{14}$ est alkyle mono-insaturé ou alkylthio, k soit 1, 2 ou 3 ;

$R^6$ est dihydroxyalkyle, dialcoxyalkyle, alcoxyalcoxyalkyle, haloalkyle, (haloalcoxy)alkyle ou alkyle substitué par un noyau saturé à 5 à 6 membres comprenant 1-2 atomes d'oxygène en tant que membres du noyau où les atomes de carbone du noyau peuvent être substitués par 0-2 substituants alkyles ;

$R^7$ et $R^8$ sont indépendamment $R^6$, H, alkyle, hydroxyalkyle, alcoxyalkyle, aminoalkyle, alkylaminoalkyle, dialkylaminoalkyle ou arylalkyle, $R^7$ et $R^8$, avec l'azote auquel ils sont attachés, forment un noyau à 5-7 membres, où l'un des 4-6 membres du noyau comprenant $R^7$ et $R^8$ peut être un atome d'azote, un atome d'azote alkyle-substitué ou un atome d'oxigène, et où le noyau peut être substitué sur les atomes de carbone du noyau par des substituants choisis parmi des groupes alkyle et hydroxy ;

$R^9$ est hydrogène, alkyle, carboxyalkyle, mercaptoalkyle, alkylthioalkyle, aminoalkyle, hydroxyalkyle, phénylalkyle, hydroxyphénylalkyle, guanidinoalkyle, imidazolylalkyle, indolylalkyle ou carbamoylalkyle ;

n est 0-2 ;

m et k sont indépendamment 0-3 ;

q est 1-4 ;

X est une liaison, –O–, –S–, ou $-CH_2-$ ;

Q est hydrogène ou $R^{10}CO-$ ;

$R^{10}$ est alkyle, hydroxyalkyle, alcoxyalkyle, dialkylaminoalkyle, $Y^2-C_6H_4$-alkyle, alcoxy, $Y^2-C_6H_4-$, naphtyle, furyle, thiényle ou pyridyle ;

Y, $Y^1$ et $Y^2$ représentent indépendamment un ou plusieurs substituants choisis parmi H, alkyle, cycloalkyle, alcoxy, OH, F, Cl, Br, CN, $-CH_2NH_2$, $-CO_2H$, $-CO_2$-alkyle, $-CONH_2$ et phényle ;

et leurs sels d'addition acceptables en pharmacie.

2. Composé selon la revendication 1 où $R^2$ est $R^{14}(CH_2)_kS(O)_{0-2}(CH_2)_q-$.

3. Composé selon la revendication 2 où $R^{14}$ est alkyle inférieur mono-insaturé ou alkylthio.

4. Composé selon l'une quelconque des revendications 1 à 3 où $R^1$ est $Y-C_6H_4-$.

5. Composé selon l'une quelconque des revendications 1 à 4 où Q est hydrogène ou $R^{10}CO-$ où $R^{10}$ est alkyle ou phényle.

6. Composé selon l'une quelconque des revendications 1 à 5 où $R^3$ est $-OR^7$ ou $-NR^7R^8$,

7. Composé selon la revendication 1 qui est

N-[3-benzoylthio-2(S)-benzylpropionyl]-S-allyl-(R)-cystéinamide ;

N-[3-benzoylthiométhyl-2(S)-benzylpropionyl]-S-méthylthiométhyl-(R)-cystéinamide ;

N- [3-acétylthio-2(S)-benzylpropionyl]-S-allyl-(R)-cystéinamide ;

N-[3-acétylthio-2(R)-benzylpropionyl]-S-allyl-(R)-cystéinamide ;

N- [3-acétylthio-2(S)-benzylpropionyl)-S-méthylthiométhyl-(R)-cystéinamide ;

N- [3-acétylthio-2(R)-benzylpropionyl)-S-méthylthiométhyl-(R)-cystéinamide ;

Ester éthylique de N-[3-acétylthio-2(S)-benzylpropionyl]-S-méthylthiométhyl-(R)-cystéine ;

Ester éthylique de N-[3-acétylthio-2(R)-benzylpropionyl]-S-méthylthiométhyl-(R)cystéine ;

Ester 2-(2-chloroéthoxy)éthylique de N-[2(S)-acétylthiométhyl-3-phénylpropionyl)-S-allyl-(R)-cystéine ;

Ester 2(2-chloroéthoxy)éthylique de N-[2(R)-acéthylthiométhyl-3-phénylpropionyl)-S-allyl-(R)-cystéine ;

Ester 2-(2-chloroéthoxy)éthylique de N-[2(S)-benzoylthio-3-phénylpropionyl-S-allyl-(R)-cystéine ;

Ester 2-(2-chloroéthoxy)éthylique de N-[2(S)-benzoylthio-3-phénylproprionyl)-S-méthylthiométhyl-(R)-cystéine ; et

β-benzyl N-[2(R,S)-mercaptométhyl-3-phénylpropionyl]-(S)-aspartate.

8. Composition pharmaceutique comprenant comme ingrédient actif un composé selon l'une quelconque des revendications 1 à 7.

9. Utilisation d'un composé selon l'une quelconque des revendications 1 à 7 pour la préparation d'une composition pharmaceutique pour le traitement de l'hypertension et/ou de l'asystolie congestive.

10. Procédé pour la préparation d'un composé de la formule I tel que défini à la revendication 1,où le composé est préparé par un procédé approprié choisi parmi les procédés A et B qui suivent et où Q, $R^1$, $R^2$, $R^3$ et n sont définis à la revendication 1, comprenant une protection appropriée :

Procédé A : condensation d'un acide 3-thiopropionique de formule IV ou son dérivé réactif, avec une amine de formule V

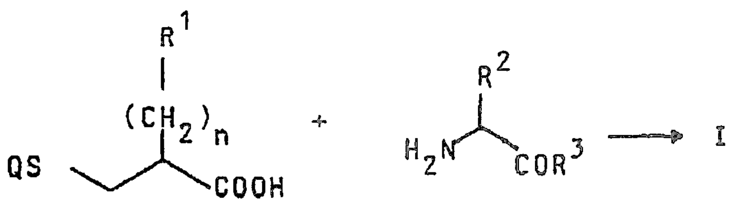

IV V

Procédé B : pour des composés de formule I où $R^3$ est $-NR^7R^8$, condensation d'un acide (3-thiopropionyl-)amino de formule VI ou son dérivé réactif, avec une amine de formule VII

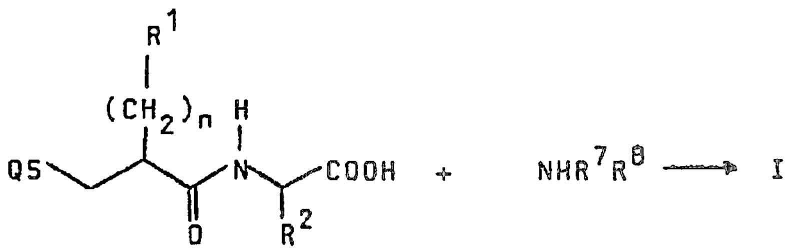

VI VII

où les deux procédé A et procédé B sont suivis de l'isolement de l'isomère préféré si on le souhaite et l'élimination des groupes protecteurs si nécessaire pour donner les produits souhaités et si on le souhaite la préparation d'un sel.

**Revendications pour l'Etat Contractant : ES**

1. Procédé pour la production d'un composé ayant la formule de structure

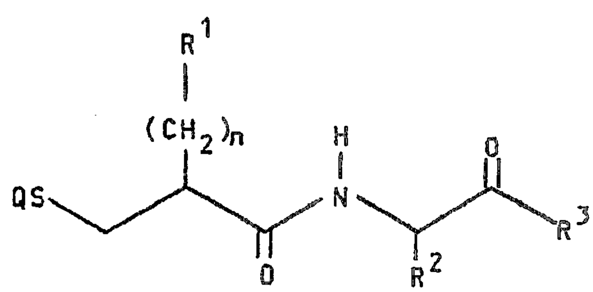

où $R^1$ est $Y-C_6H_4-$, $Y-C_6H_4S-$, $Y-C_6H_4O-$,

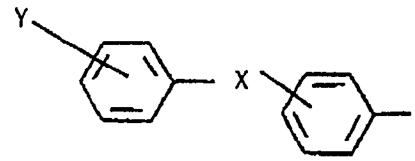

α-naphtyle, β-naphtyle, furyle, thiényle, benzofuryle, benzothiényle, $H_2N(CH_2)_m$–, diphénylméthyle, ou

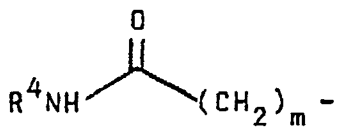

$R^2$ est $R^{14}(CH_2)_kS(O)_{0-2}(CH_2)_q$– ou $R^6OCO(CH_2)_q$– ;
$R^3$ est –$OR^7$, –$NR^7R^8$,

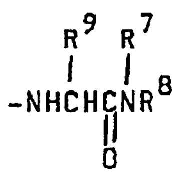 , 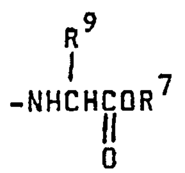 ou 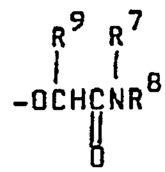

$R^4$ est hydrogène, alkyle ou $Y^1$-$C_6H_4$– ;
$R^{14}$ est alkyle inférieur mono-insaturé, hydroxy, alcoxy, ou alkylthio à condition que quand $R^{14}$ est hydroxy ou alcoxy, k soit 2 ou 3 et quand $R^{14}$ est alkyle mono-insaturé ou alkylthio, k soit 1, 2 ou 3 ;
$R^6$ est dihydroxyalkyle, dialcoxyalkyle, alcoxyalcoxyalkyle, haloalkyle, (haloalcoxy)alkyle ou alkyle substitué par un noyau saturé à 5 ou 6 membres comprenant 1-2 atomes d'oxygène en tant que membres du noyau où les atomes de carbone du noyau peuvent être substitués par 0-2 substituants alkyles ;
$R^7$ et $R^8$ sont indépendamment $R^6$, H, alkyle, hydroxyalkyle, alcoxyalkyle, aminoalkyle, alkylaminoalkyle, dialkylaminoalkyle ou arylalkyle, $R^7$ et $R^8$, avec l'azote auquel ils sont attachés, forment un noyau à 5-7 membres, où l'un des 4-6 membres du noyau comprenant $R^7$ et $R^8$ peut être un atome d'azote, un atome d'azote alkyle-substitué ou un atome d'oxygène, et où le noyau peut être substitué sur les atomes de carbone du noyau par des substituants choisis parmi des groupes alkyle et hydroxy ;
$R^9$ est hydrogène, alkyle, carboalkyle, mercaptoalkyle, alkylthioalkyle, aminoalkyle, hydroxyalkyle, phénylalkyle, hydroxyphénylalkyle, guanidonoalkyle, imidazolylalkyle, indolylalkyle ou carbamoylalkyle ;
n est 0-2 ;
m et k sont indépendemment 0-3 ;
q est 1-4 ;
X est une liaison, –O–, –S–, ou –$CH_2$– ;
Q est hydrogène ou $R^{10}CO$– ;
$R^{10}$ est alkyle, hydroyalkyle, alcoxyalkyle, dialkylaminoalkyle, $Y^2$-$C_6H_4$-alkyle, alcoxy, $Y^2$-$C_6H_4$–, naphtyle, furyle, thiényle ou pyridyle ;
Y, $Y^1$ et $Y^2$ représentent indépendamment un ou plusieurs substituants choisis parmi H, alkyle, cycloalkyle, alcoxy, OH, F, Cl, Br, CN, –$CH_2NH_2$, –$CO_2H$, –$CO_2$-alkyle, –$CONH_2$ et phényle ;
et leurs sels d'addition acceptables en pharmacie, où le composé est préparé par un procédé approprié choisi dans les procédés A et B suivants, et où Q, $R^1$, $R^2$, $R^3$ et n sont définie à la revendication 1, comprenant une protection appropriée :

Procédé A : condensation d'un acide 3-thiopropionique de formule IV ou son dérivé réactif, avec une amine de formule V

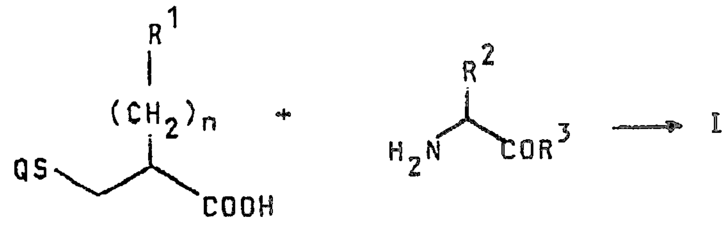

IV V

Procédé B : pour des composés de formule I où $R^3$ est $-NR^7R^8$, condensation d'un acide (3-thiopropionyl) amino de formule VI ou son dérivé réactif avec une amide de formule VII

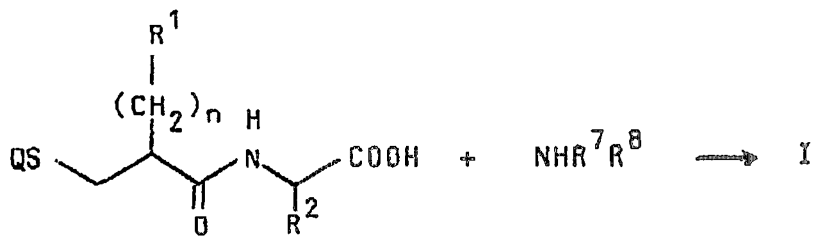

VI VII

où les deux procédé A et procédé B sont suivis de l'isolement de l'isomère préféré si on le souhaite et l'élimination des groupes protecteurs si nécessaire pour dnnner les produits souhaités et si on le souhaite la préparation d'un sel.

2. Procédé selon la revendication 1 pour la production d'un composé où $R^2$ est $R^{14}(CH_2)_k\,S(O)_{0-2}(CH_2)_q-$.

3. Procédé selon la revendication 2 pour la production d'un composé où $R^{14}$ est alkyle inférieur mono-insaturé ou alkylthio.

4. Procédé selon les revendications 1 à 3 pour la production d'un composé où $R^1$ est $Y-C_6H_4$.

5. Procédé selon les revendications 1 à 4 pour la production d'un composé où 4 est hydrogène ou $R^{10}CO-$ où $R^{10}$ est alkyle ou phényle.

6. Procédé selon les revendications 1 à 5 pour la production d'un composé où $R^3$ est $-OR^7$ ou $-NR^7R^8$.

7. Procédé selon la revendication 1 pour la production d'un composé qui est
N-[3-benzoylthio-2(S)-benzylpropionyl]-S-allyl-(R)-cystéinamide ;
N- [3-benzoylthiométhyl-2(S)-benzylpropionyl]-S-méthylthiométhyl-(R)-cystéinamide ;
N-[3-acétylthio-2(S)-benzylpropionyl)-S-allyl-(R)-cystéinamide :
N-[3-acétylthio-2(R)-benzylpropionyl]-S-allyl-(R)-cystéinamide :
N-[3-acétylthio-2(S)-benzylpropionyll]-S-méthylthiométhyl-(R)-cystéinamide ;
N- [3-acétylthio-2(R)-benzylpropionyl(-5-méthylthiométhyl-(R)-cystéinamide ;
Ester éthylique de N-(3-acétylthio-2(S)-benzylpropionyl]-S-méthylthiométhyl-(R)-cystéine ;
Ester éthylique de N-(3-acélylthio-2(R)-benzylpropionyl]-S-méthylthiométhyl-(R)-cystéine ;
Ester 2-(2-chloroéthoxy)éthylique de N-(2-(S)-acétylthiométhyl-3-phénylproprionyl-S-allyl-(R)-cystéine ;
Ester 2(2-chloroéthoxy)éthylique de N-(2(R)-acétylthiométhyl-3-phénylpropionyl)-S-allyl-(R)-cystéine ;
Ester 2-(2-chloroéthoxy)éthylique de N-[2(S)-benzoylthio-3-phénylpropionyl)-S-allyl-(R)-cystéine ;
Ester 2-(2-chloroéthoxy)éthylique de N-[2(S)-benzoylthio-3-phénylpropionyl)-S-méthylyhiométhyl-(R)-cystéine ; et
β-benzyl N-[2(R,S)-mercaptométhyl-3-phénylpropionyl]-(S)-aspartate.

8. Procédé pour la production d'une composition pharmaceutique comprenant comme ingrédient actif un composé produit selon un procédé tel que revendiqué dans l'une des revendications 1 à 7 où l'ingrédient actif est mélangé à un véhicule acceptable en pharmacie.

9. Utilisation d'un composé produit selon un procédé tel que revendiqué dans l'une des revendications 1 à 7 pour la préparation d'une composition pharmaceutique pour le traitement de l'hypertension et/ou de la systolie congestive.